# EUROPEAN PATENT APPLICATION

(11) **EP 3 284 817 A1**
(43) Date of publication of application: **21.02.2018**
(21) Application number: 16306065.0
(22) Date of filing: 18.08.2016
(51) Int. Cl.: C12N 5/071, G01N 33/50

(54) **HUMAN SEBOCYTE PRECURSOR CELLS, HUMAN SEBOCYTES AND IN VITRO METHODS FOR OBTAINING THE SAME FROM HUMAN INDUCED PLURIPOTENT STEM CELLS (HIPSC)**

(71) Applicant: Phenocell, 91058 Evry Cedex (FR)
(72) Inventor: ONTENIENTE, Brigitte, 94600 CHOISY-LE-ROI (FR); MARUOTTI, Julien, 91250 SAINTRY-SUR-SEINE (FR)
(74) Representative: Nony

(57) **Abstract**

One aspect of the invention relates to an *in vitro* method for obtaining hiPSC-derived sebocyte precursor cells comprising the steps of:
a) culturing, under feeder layer-free conditions, human induced pluripotent stem cells (hiPSC) in a sebocyte basal medium complemented with a combination of (i) retinoic acid (RA), (ii) bone morphogenetic protein 4 (BMP4) and (iii) epidermal growth factor (EGF), during a period of time ranging from about 5 days to about 15 days, so as to generate multipotent skin precursor cells,
b) culturing, under feeder layer-free conditions, the multipotent skin precursor cells obtained at step a) in a sebocyte differentiation basal medium, during a period of time ranging from about 5 days to about 15 days, so as to generate hiPSC-derived sebocyte precursor cells, and
c) collecting the hiPSC-derived sebocyte precursor cells obtained at step b).

## Description

### FIELD OF THE INVENTION

The present invention relates to an *in vitro* method for generating human sebocyte precursor cells from human induced pluripotent stem cells (hiPSC) and an *in vitro* method for generating human sebocyte cells from said human sebocyte precursor cells.

Both cell populations, the hiPSC-derived sebocyte precursor cells and the hiPSC-derived mature sebocyte cells, are characterized by expression of KRT7 and MUC1 markers, are able to promote lipogenesis after induction by linoleic acid (LA) and dihydrotestosterone (DHT), and display a lipid content that is very similar to *in vitro* cultured human sebocyte cells isolated from human individuals.

### BACKGROUND OF THE INVENTION

The skin of mammals is a complex organ that comprises at least three functionally distinct layers, i.e. the epidermis, the dermis and the hypodermis (also referred as subcutis).

Among many other structures, the dermis comprises sebaceous glands, which are microscopic exocrine and holocrine glands.

The sebaceous glands are spread in humans throughout the skin except in the palms of the hands and soles of the feet. They are naturally found in greatest abundance on the face and the scalp. Sebaceous glands can usually be found in hair-covered areas where they are connected to hair follicles, but also in non-haired areas of the body, such as lips and eyelids.

The sebaceous glands secrete an oily and waxy substance called sebum, which is made of triglyceride oils, wax, squalene, metabolites of fat-producing cells and cellular debris. Sebum naturally achieves protection and waterproofing of hairs and skin, and keeps them from dryness, brittleness, and cracking. Sebum has also been reported as inhibiting the growth of microorganisms on skin.

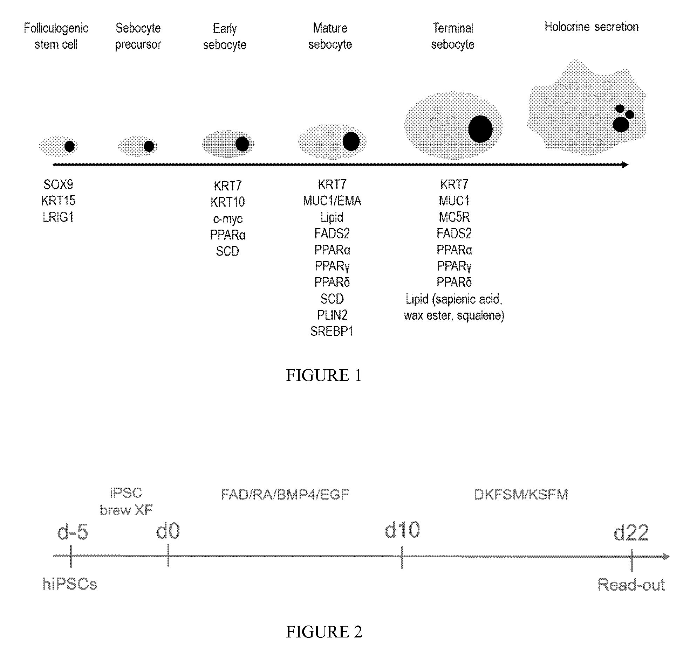

Sebocytes are the specialized cells responsible for sebum production in the sebaceous gland. As a result of accumulation of large quantities of sebum, sebocytes burst out at the end of their physiological maturation, and sebum is released into the sebum canal (Figure 1).

Aside physiological sebum production, dysfunctions of the sebaceous glands may result in excessive production of sebum, rendering the skin greasy in appearance. This skin condition is often referred to as seborrhea or hyper-seborrhea.

During puberty or under some contraceptive treatments, hormonal changes may promote an excessive production of sebum, which is often associated with bacteria proliferation and inflammation, this condition being referred as *acne vulgaris*.

In addition, dermatitis of sebaceous areas, or seborrheic dermatitis, is an inflammatory disorder that affects around 3% of the population. Atrophy of the sebaceous gland is observed in atopic dermatitis and psoriasis.

The occurrence of hypo- or hyper-seborrheic dysfunctions explains the major research interest in dermatology for unravelling the function of the sebaceous gland under normal and pathological conditions. This includes investigations of transcriptional mechanisms that regulate lipid metabolism and inflammatory responses of sebocytes (Toth et al., 2011).

Identification of specific regulatory mechanisms and of key actors in these processes is essential to fully understand the normal physiological function of the sebaceous gland and to analyse pathological modifications. A particularly relevant area of research is the regulation of gene expression by transcription factors that respond to changes in fatty molecules and therefore link metabolic processes to gene regulation.

Adult primary sebocytes are of special interest, in particular as research tools for a variety of applications in acne, seborrhea and other sebaceous gland-related diseases studies in skin biology/dermo-cosmetic programs.

Initially, studies have been performed with sebocytes from animals (mainly rodents). However, sebum composition varies from species to species and only humans suffer from acne. Therefore, animal sources, for example rat preputial sebocytes (Pochi, 1985), have limited relevance to provide *in vitro* models to study human skin physiological pathways or skin diseases or conditions related to dysfunctions of the sebaceous gland.

To date, two sources of human sebocyte cells have been used for *in vitro* studies, namely sebocytes from human skin (primary source) or sebocytes from immortalized cell lines.

Sebocytes from human donors are obtained after digestion of the skin and culture in specific media. Deriving sebocytes from primary cultures is a tedious process, with poor yield as these cells do not amplify very efficiently (Xia et al., 2009). In addition, skin sources are most commonly leftover explants from surgical procedures, which implies little, if any, information on the health status of the donor, and high lot-to-lot variability. In total, sebocytes from primary sources are expensive, their availability is limited and quality varies.

In an effort to increase the availability of sebocytes, academic research groups have produced immortalized cell lines from primary cells. One of the most common sebocyte immortalized cell line is SZ95 (Zouboulis et al., 1999). Although these cells recapitulate the expression of specific markers and are functional as evidenced by their response to linoleic acid (LA) or the production of specific sebum components, they are genetically-modified organisms and display an altered karyotype (for example, SZ95 has over 60 chromosomes). In addition, immortalized sebocytes lack androgen receptors (Barrault et al., 2015), they cannot respond to testosterone to promote lipogenesis (Chen et al., 2003), and do not provide a suitable *in vitro* model for studying acne.

Most recently, putative sebocytes were obtained after differentiation of human induced pluripotent stem cells (hiPSC; Yang et al., 2014). These sebocyte cells expressed KRT7, PPAR-α and LPL by quantitative PCR, and displayed lipid droplets in their cytoplasm. However, the expression of MUC1, a more mature sebocyte marker, was not reported, nor was cell response to LA and DHT demonstrated. Importantly, the differentiation into putative sebocyte cells was performed with a first step of embryoid body formation, followed by culture on a monolayer of animal feeder cells. Not only is such an approach not adapted to large-scale production, but it also results in a putative sebocyte population contaminated with animal cells and with a purity degree incompatible with industrial standards.

Therefore, there is an urgent need in the art for a method to produce human sebocytes without the use of contaminating animal cells and with increased purity and yield, which could be suitable for providing relevant *in vitro* models for disease modelling and definition of innovative therapeutic approaches.

### SUMMARY OF THE INVENTION

The present invention overcomes a major deficiency in the art in providing functional human sebocytes in large-scale, high purity and with defined genetic and clinical background. The present invention relates to the field of stem cell biology, in particular the linage specific differentiation of pluripotent or multipotent stem cells, which can include, but is not limited to, human induced pluripotent stem cells (hiPSC), human embryonic stem cells (hESC), or any other cell capable of lineage specific differentiation.

One aspect of the invention relates to an *in vitro* method for obtaining hiPSC-derived sebocyte precursor cells comprising the steps of:
a) culturing, under feeder layer-free conditions, human induced pluripotent stem cells (hiPSC) in a sebocyte basal medium complemented with a combination of (i) retinoic acid (RA), (ii) bone morphogenetic protein 4 (BMP4) and (iii) epidermal growth factor (EGF), during a period of time ranging from about 5 days to about 15 days, so as to generate multipotent skin precursor cells,
b) culturing, under feeder layer-free conditions, the multipotent skin precursor cells obtained at step a) in a sebocyte differentiation basal medium, during a period of time ranging from about 5 days to about 15 days, so as to generate hiPSC-derived sebocyte precursor cells, and
c) collecting the hiPSC-derived sebocyte precursor cells obtained at step b).

Another aspect of the invention relates to hiPSC-derived sebocyte precursor cell population obtained by the *in vitro* method as described herein.

Another aspect of the invention relates to an *in vitro* method for obtaining hiPSC-derived sebocytes comprising the step of a) of culturing, under feeder layer-free conditions, the hiPSC-derived sebocyte precursor cells obtained by the method as described above, in a sebocyte basal medium complemented by an inhibitor of the Small Mothers Against Decapentaplegic (SMAD) protein signalling pathway, during a period of time of about 2 days to about 7 days.

Another aspect of the invention relates to hiPSC-derived sebocytes obtained by an *in vitro* method as described herein.

One aspect of the invention relates to the use of hiPSC-derived sebocyte precursor cells and/or hiPSC-derived sebocytes as described herein, for the preparation of an *in vitro* human model of hypo-seborrheic human skin or hyper-seborrheic human skin.

Another aspect of the invention relates to the use of hiPSC-derived sebocyte precursor cells and/or hiPSC-derived sebocytes as described herein, for the preparation of an *in vitro* human model of 3D skin epithelium.

In still other aspect, the invention relates to the use of hiPSC-derived sebocytes as described herein, for the preparation of an *in vitro* human model of testosterone-sensitive cells.

In a further aspect, the invention also relates to the use of hiPSC-derived sebocytes as described herein, for the preparation of an *in vitro* human model of linoleic acid-sensitive cells.

In another aspect, the invention also relates to the use of hiPSC-derived sebocytes as described herein, as a tool for screening a compound suitable for the treatment and/or the prevention of dysfunctions of a sebaceous gland.

In a further aspect, the invention relates to the use of hiPSC-derived sebocytes as described herein, as a tool for screening a compound suitable for the treatment and/or the prevention of hypo-seborrheic or hyper-seborrheic conditions of the human skin.

### LEGENDS OF THE FIGURES

**Figure 1****:** Scheme illustrating the maturation process of sebocytes up to the holocrine secretion, and the variation of markers expression during the said maturation, i.e. from the follilogenic stem cell state to the terminal sebocyte state.
**Figure 2****:** Scheme illustrating the overall protocol for the generation of sebocyte precursor cells (at day 22 (d22)) starting from hiPSC. From d-5 to d0, hiPSC are cultured in hiPSC standard feeder-free medium (iPSC brew XF). At d0, the medium is removed and replaced by a basal medium (FAD medium complemented with FBS, human insulin, human recombinant EGF, hydrocortisone, cholera toxin B subunit and adenine), further complemented with RA, BMP4 and EGF. At d10, the RA-, BMP4- and EGF-complemented basal medium is removed and replaced by a 1:1 (vol:vol) DK-SFM:K-SFM medium. At d22, sebocyte precursor cells are obtained and analysed for determining the expression of selected markers.

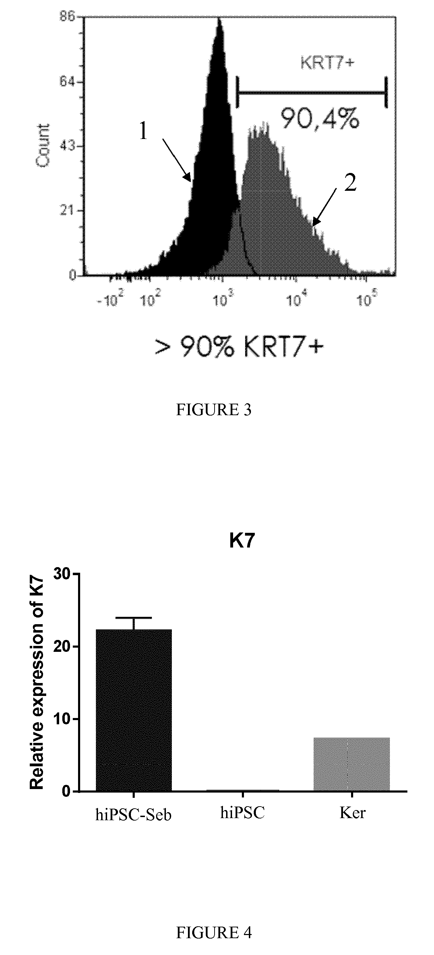

**Figure 3****:** Plot illustrating the phenotypic analysis by flow cytometry of hiPSC-derived sebocyte precursor cells obtained as described in Figure 2 for the KRT7 marker. Curve 1 represents hiPSC and curve 2 represents sebocyte precursor cells.
**Figure 4****:** Plot illustrating the relative expression of the KRT7 marker ("K7") in hiPSC-derived sebocyte precursor cells (hiPSC-Seb), obtained accordingly to Figure 2, hiPSC and primary keratinocytes ("Ker").

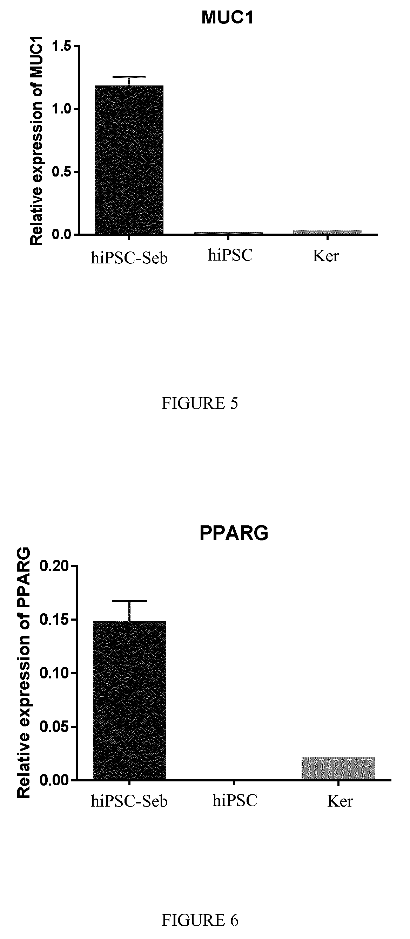

**Figure 5****:** Plot illustrating the relative expression of the MUC1 marker in hiPSC-derived sebocyte precursor cells (hiPSC-Seb), obtained accordingly to Figure 2. "Ker" is as defined in Figure 4.
**Figure 6****:** Plot illustrating the relative expression of the peroxisome-activated receptor gamma (PPARG) marker in hiPSC-derived sebocyte precursor cells (hiPSC-Seb), obtained accordingly to Figure 2. "Ker" is as defined in Figure 4.

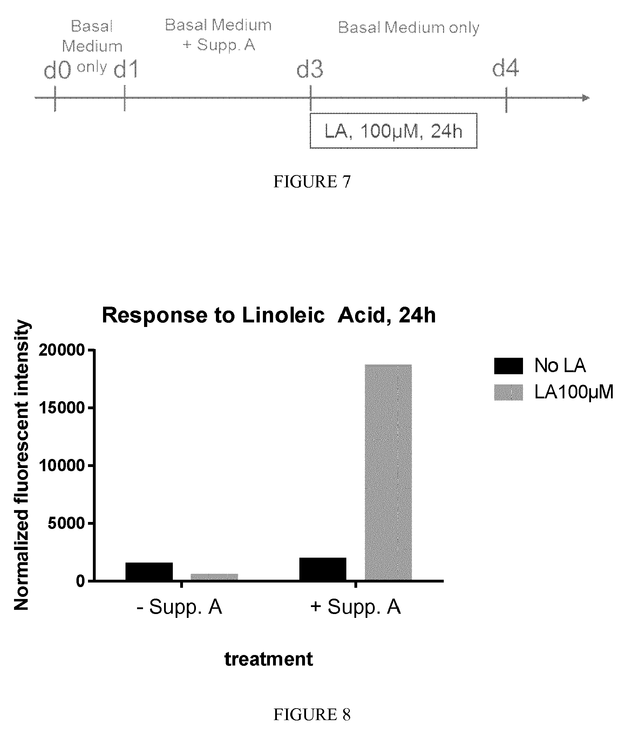

**Figure 7****:** Scheme illustrating the overall protocol for assessing the linoleic acid (LA) response of hiPSC-derived sebocytes cultured from d1 to d3 in a basal medium complemented with Supplement A (SB431542). At d3 and as indicated in the box, sebocytes are further processed in the absence or the presence of 100 µM of LA for 24 h, in order to assess induction of the lipogenesis pathway.
**Figure 8****:** Plot illustrating the LA response of hiPSC-derived sebocytes cultured in the absence (-Supp. A) or the presence (+Supp. A) of Supplement A, as described in Figure 7, and further processed for 24 h without (black bars) or with 100 µM LA (gray bars). The normalized fluorescent intensity is correlated with cellular lipogenesis.

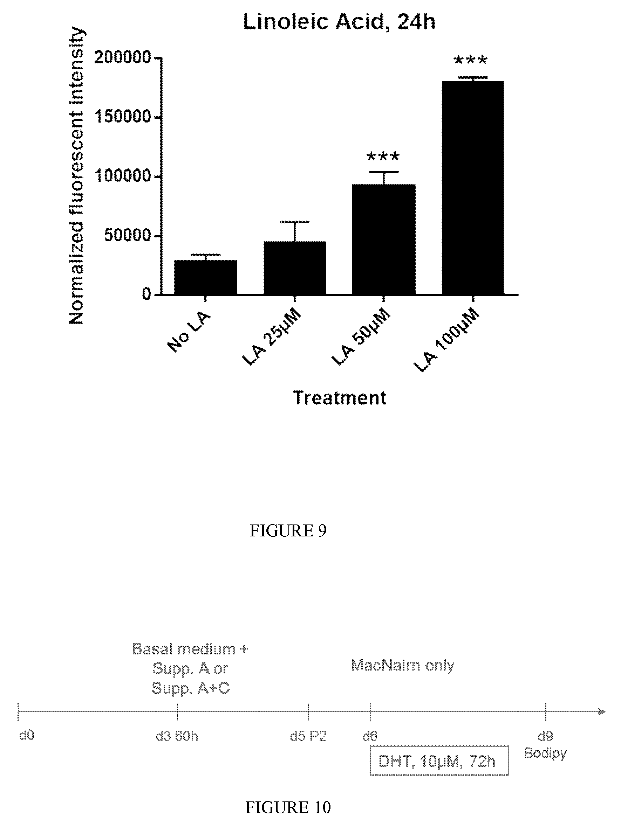

**Figure 9****:** Plot illustrating the LA response of hiPSC-derived sebocytes cultured in the presence of Supplement A (+Supp. A), as described in Figure 7, and further processed for 24 h without or with increasing amounts (25 µM, 50 µM and 100 µM) of LA. The normalized fluorescent intensity is correlated with cellular lipogenesis.
**Figure 10****:** Scheme illustrating the overall protocol for assessing dihydrotestosterone (DHT) response of hiPSC-derived sebocytes cultured in basal medium supplemented with SB431542 ("supplement A") or with SB431542 and GW0742 ("supplement A+C"). At d6, sebocytes are further processed in the absence or presence of 10 µM of DHT for 72 h.

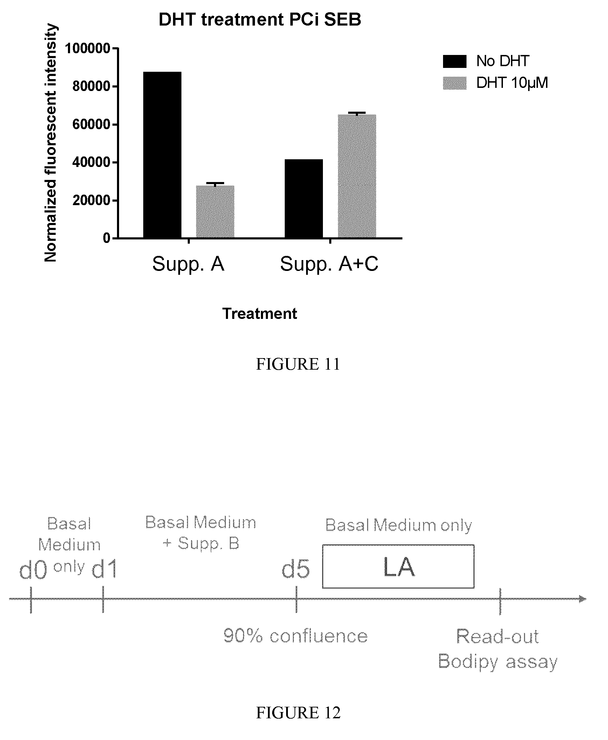

**Figure 11****:** Plot illustrating the DHT response at d6 of hiPSC-derived sebocytes (PCI SEB) cultured as described in Figure 10, in the absence (black bars) or presence (gray bars) of 10 µM DHT. The normalized fluorescent intensity is correlated with cellular lipogenesis.
**Figure 12****:** Scheme illustrating the overall protocol for assessing LA response of hiPSC-derived sebocytes cultured in basal medium complemented with Supplement B (cyclopamine) from d1 to d5. As indicated, hiPSC-derived sebocytes from d5 are further processed for 24 h without or with varying amounts of LA for 24 h.

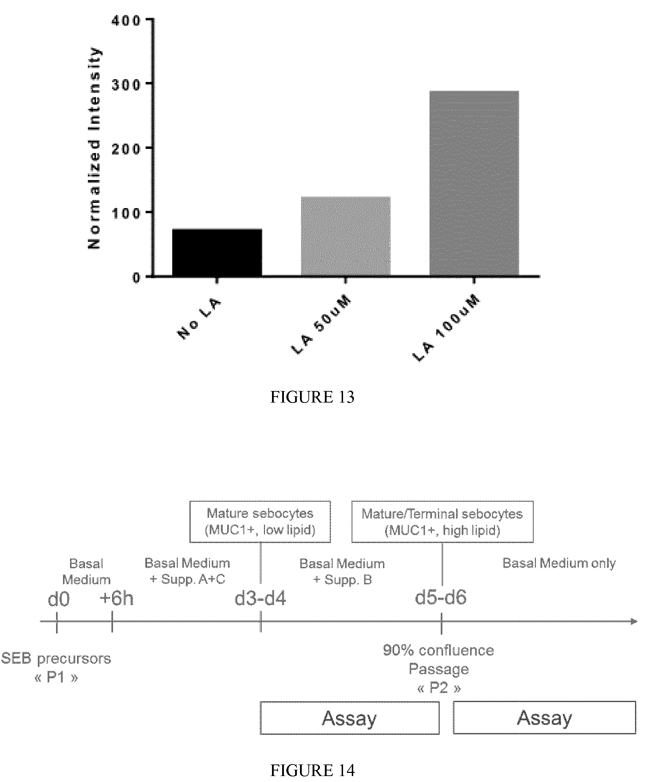

**Figure 13****:** Plot illustrating the LA response of hiPSC-derived sebocytes cultured in the presence of supplement B, as described in Figure 12, without (black bar) or with (gray bars) increasing amounts of LA (50 µM and 100 µM) for 24 h. The normalized fluorescent intensity is correlated with cellular lipogenesis.
**Figure 14****:** Scheme illustrating the overall protocol for assessing the phenotypic characterisation at d5-6 of hiPSC-derived sebocytes cultured sequentially for 3/4 days in basal medium complemented with supplement A+C and for 2 extra days in basal medium complemented with supplement B.

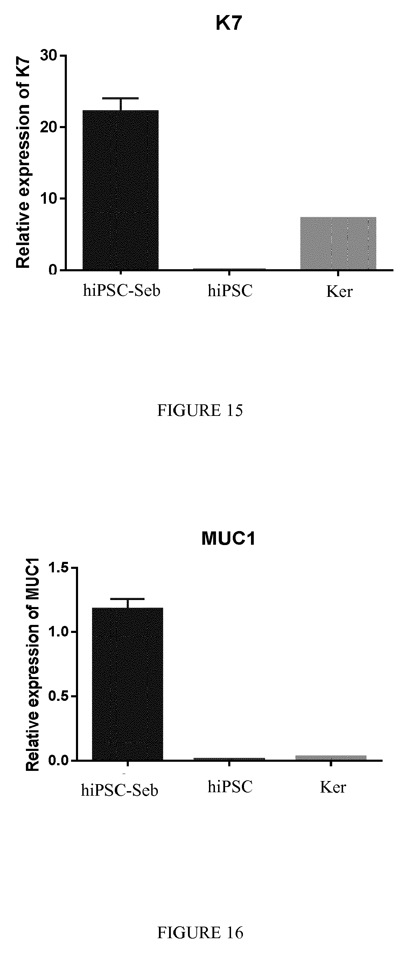

**Figure 15****:** Plot illustrating the relative expression of KRT7 marker ("K7") at d5-6 in hiPSC-derived sebocytes (hiPSC-Seb), obtained as described in Figure 14, hiPSC and primary keratinocytes (Ker).
**Figure 16****:** Plot illustrating the relative expression of MUC1 marker at d5-6 in hiPSC-derived sebocytes (hiPSC-Seb), obtained as described in Figure 14, hiPSC and primary keratinocytes (Ker).

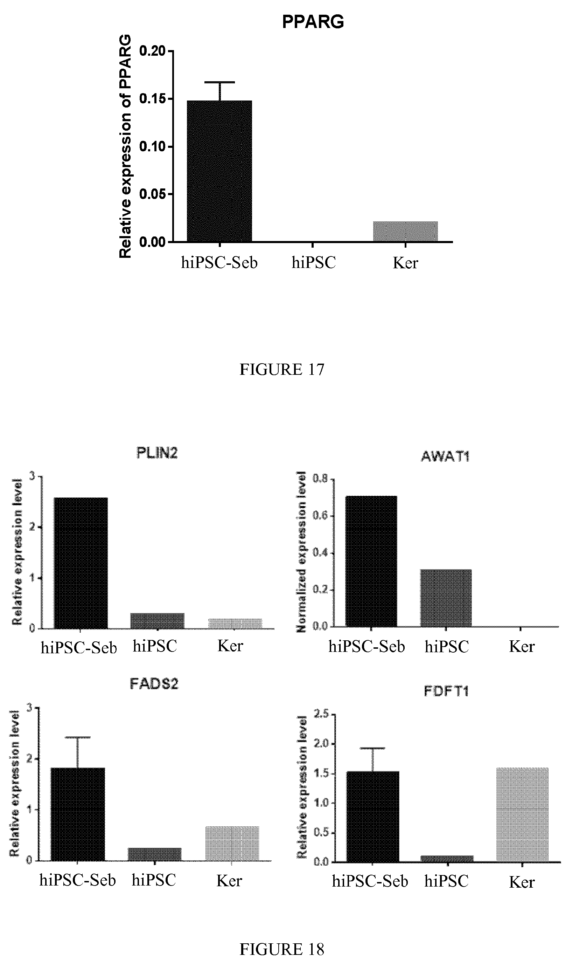

**Figure 17****:** Plot illustrating the relative expression of PPARG marker at d5-6 in hiPSC-derived sebocytes (hiPSC-Seb), obtained as described in Figure 14, hiPSC and primary keratinocytes (Ker).
**Figure 18****:** Plot illustrating the relative expression of PLIN2 (left top panel), FADS2 (left bottom panel), AWAT1 (right top panel) and FDFT1 (right bottom panel) marker at d5-6 in hiPSC-derived sebocytes (hiPSC-Seb), obtained as described in Figure 14. hiPSC and primary keratinocytes (Ker) are presented for comparison.

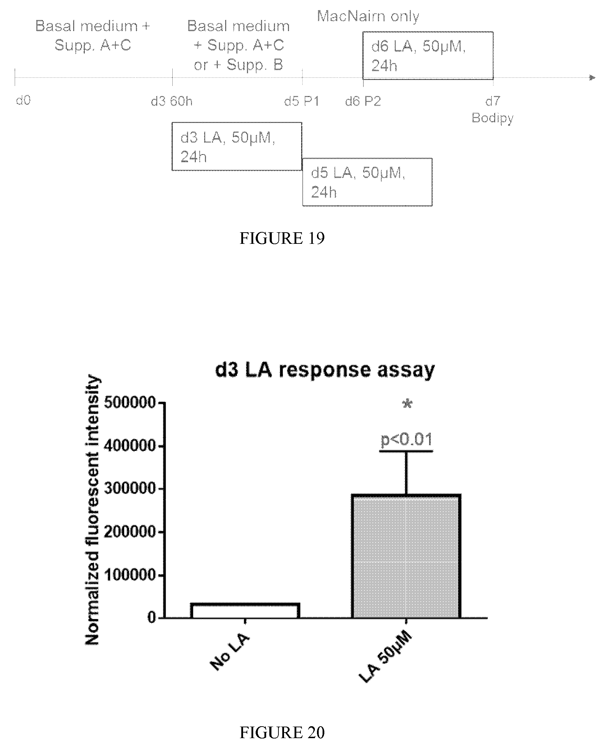

**Figure 19****:** Scheme illustrating the overall protocol for assessing the LA response of sebocytes cultured sequentially (1) for 3 days in basal medium complemented with supplement A+C and (2) for 2 days in basal medium complemented with either supplement A+C or with supplement B. Sebocytes are further processed from d3, d5 and d6 for 24 h without or with 50 µM of LA to assess lipogenesis induction.
**Figure 20****:** Plot illustrating the LA response at d3 of sebocyte cells cultured for 3 days in basal medium complemented with supplement A+C, as described in Figure 19, without (white bar) or with 50 µM LA (gray bar) . The normalized fluorescent intensity is correlated with cellular lipogenesis.

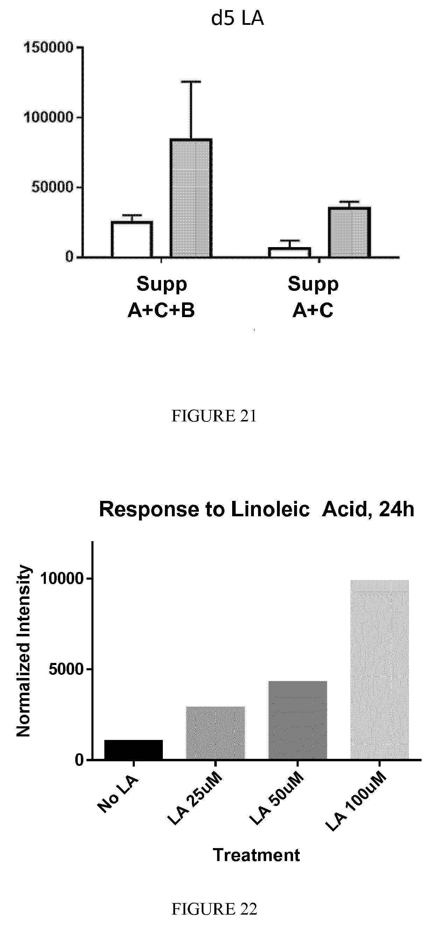

**Figure 21****:** Plot illustrating the LA response at d5 of hiPSC-derived sebocytes cultured for 5 days in basal medium complemented with supplement A+C (Supp. A+C; right panels) or cultured sequentially for 3 days in basal medium complemented with supplement A+C and for 2 days in basal medium complemented with supplement B (Supp. A+C+B; left panels), as described in Figure 19, without (white bar) or with 50 µM LA (gray bar). The normalized fluorescent intensity is correlated with cellular lipogenesis.
**Figure 22****:** Plot illustrating the LA response at d6 of hiPSC-derived sebocytes cultured sequentially for 3 days in basal medium complemented with supplement A+C and for 2 days in basal medium complemented with supplement B, as described in Figure 19, without (black bar) or with increasing amounts of LA (25 µM, 50 µM and 100 µM; gray bars). The normalized fluorescent intensity is correlated with cellular lipogenesis.

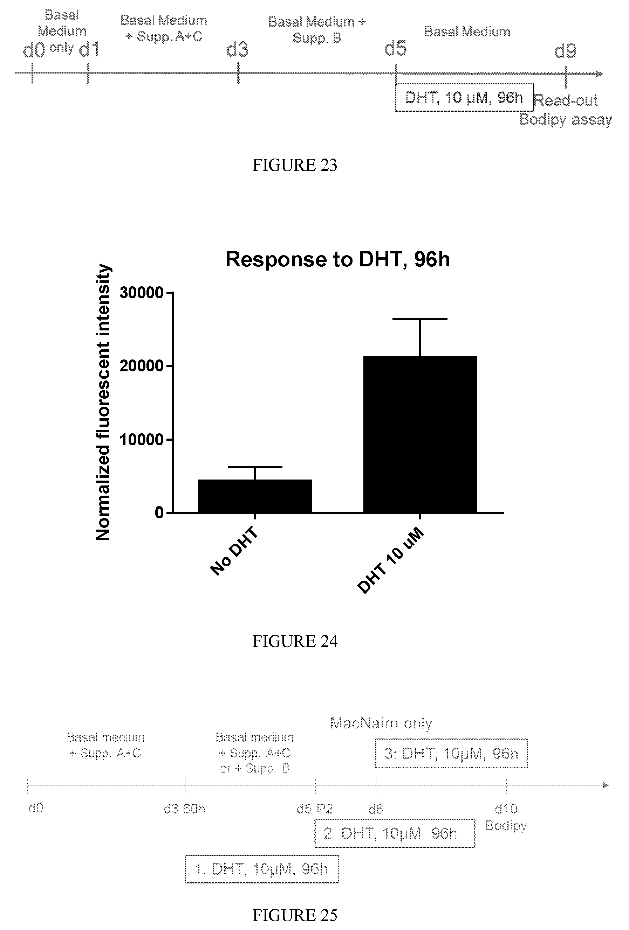

**Figure 23****:** Scheme illustrating the overall protocol for assessing the DHT response at d5 of hiPSC-derived sebocytes cultured sequentially in basal medium complemented with supplement A+C from d1 to d3, and in basal medium complemented with supplement B from d3 to d5. Sebocyte cells obtained at d5 are further processed without or with 10 µM DHT and lipogenesis is assessed after 96 h (at d9).
**Figure 24****:** Plot illustrating the DHT response of hiPSC-derived sebocytes at d5, cultured as described in Figure 23, in the absence or the presence of 10 µM of DHT. The normalized fluorescent intensity is correlated with cellular lipogenesis.
**Figure 25****:** Scheme illustrating the overall protocol for assessing the DHT response at d3, d5 and d6 of hiPSC-derived sebocytes cultured sequentially in basal medium complemented with supplement A+C for 3 days, and cultured for 2 extra days in basal medium complemented with either (1) supplement A+C or (2) supplement B. Sebocyte cells obtained after 3, 5 or 6 days are further processed without or with 10 µM DHT for 96 h. The normalized fluorescent intensity is correlated with the cellular lipogenesis.

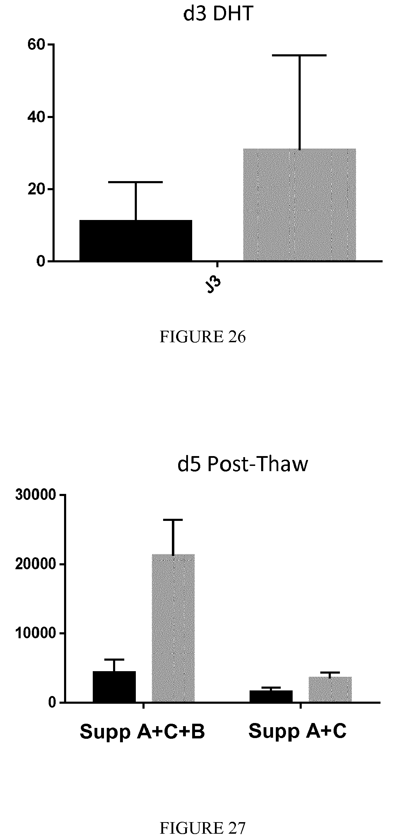

**Figure 26****:** Plot illustrating the DHT response of sebocytes at d3, cultured as described in Figure 25, in the absence (black bar) or presence (gray bar) of 10 µM DHT. The normalized fluorescent intensity is correlated with cellular lipogenesis.
**Figure 27****:** Plot illustrating the DHT response of hiPSC-derived sebocytes at d5, cultured as described in Figure 25, i.e for 5 days in the presence of supplement A+C (Supp. A+C; right panel bars) or for 3 days in the presence of supplement A+C and for 2 extra days in the presence of supplement B (Supp. A+C+B; left panel bars), in the absence (black bar) or presence (gray bar) of 10 µM DHT. The normalized fluorescent intensity is correlated with cellular lipogenesis.

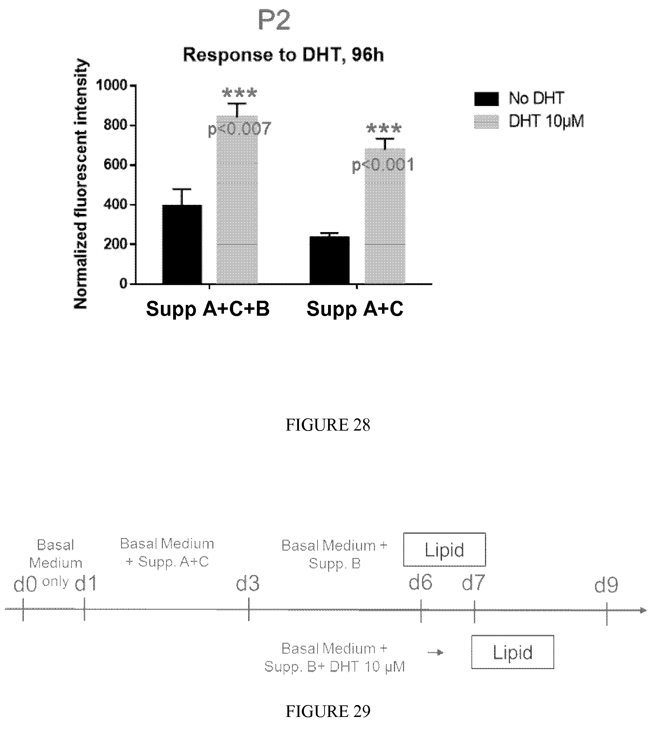

**Figure 28****:** Plot illustrating the DHT response of hiPSC-derived sebocytes at d6, cultured as described in Figure 25, i.e. for 5 days in the presence of supplement A+C (Supp. A+C; right panel bars) or for 3 days in the presence of supplement A+C and for 2 extra days in the presence of supplement B (Supp. A+C+B; left panel bars). After a 5 days culture in the conditions described above, sebocytes were cultured for 1 day in Mac Nairn medium. Sebocytes are further processed in the absence (black bar) or presence (gray bar) of 10 µM of DHT, and lipogenesis is assessed after 96 h. The normalized fluorescent intensity is correlated with cellular lipogenesis.
**Figure 29****:** Scheme illustrating the overall protocol for assessing the total lipid content at d6 of hiPSC-derived sebocytes cultured sequentially in basal medium complemented with (1) supplement A+C for 3 days, and (2) supplement B for 3 days, in the absence or the presence of 10µM DHT. Sebocyte cells obtained after 6 days are analysed for determining their total lipid content.

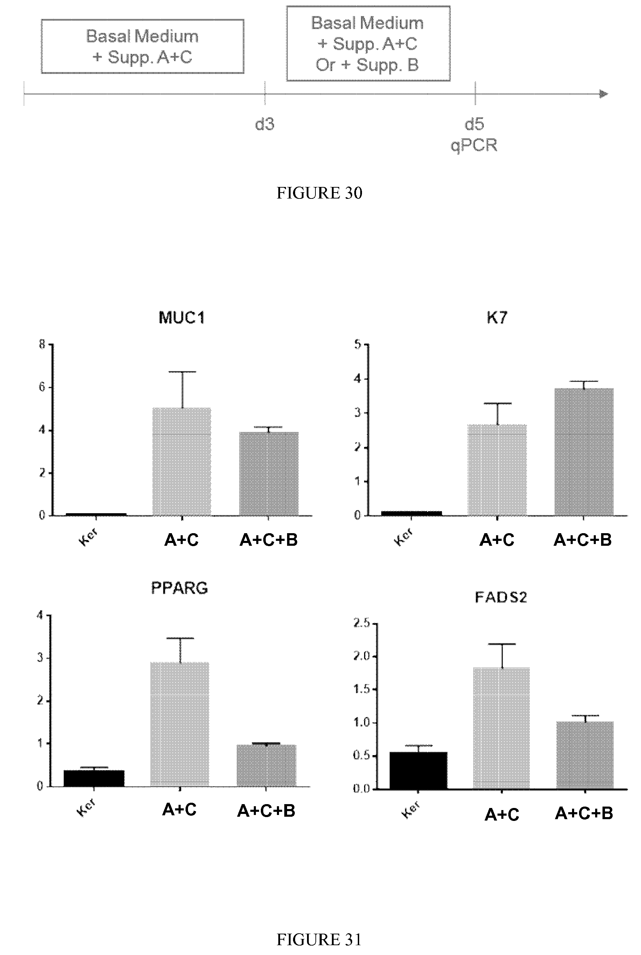

**Figure 30****:** Scheme illustrating the overall protocol for assessing the relative expression of various markers of hiPSC-derived sebocytes cultured sequentially in basal medium complemented with (1) supplement A+C for 3 days, and (2) supplement A+C or supplement B for 2 days. Sebocytes obtained after 5 days are analysed by qPCR.
**Figure 31****:** Plot illustrating the relative expression of MUC1 (left top panel), PPARG (left bottom panel), KRT7 ("K7"; right top panel) and FADS2 (right bottom panel) marker at d5 in hiPSC-derived sebocytes obtained as described in Figure 30. Primary keratinocytes ("Ker") are presented for comparison.

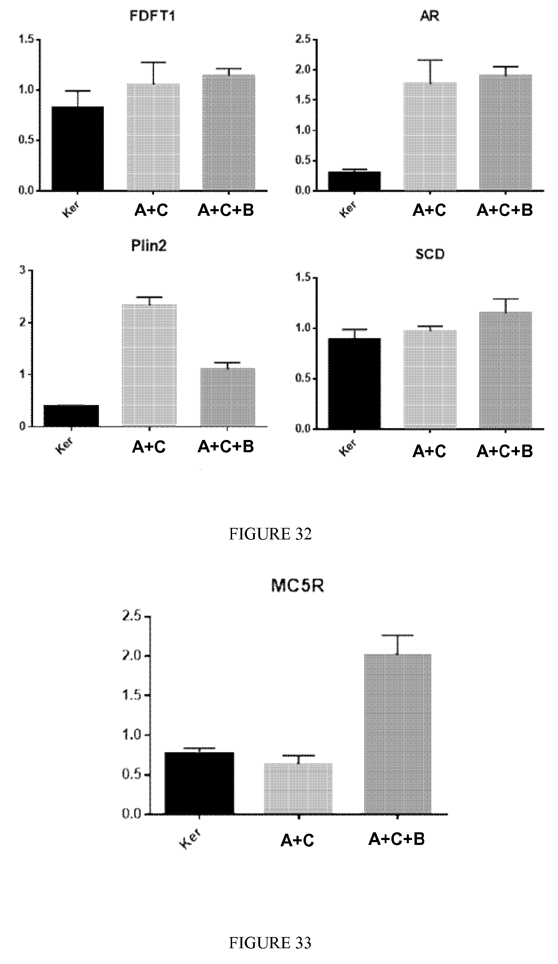

**Figure 32****:** Plot illustrating the relative expression of FDFT1 (left top panel), PLIN2 (left bottom panel), AR (right top panel) and SCD (right bottom panel) marker at d5 in hiPSC-derived sebocytes obtained as described in Figure 30. Primary keratinocytes ("Ker") are presented for comparison.
**Figure 33****:** Plot illustrating the relative expression of MC5R marker at d5 in hiPSC-derived sebocytes obtained as described in Figure 30. Primary keratinocytes ("Ker") are presented for comparison.

### DETAILED DESCRIPTION OF THE INVENTION

As illustrated by the example hereunder, the inventors have managed to generate a stable and highly homogeneous cell population, comprising hiPSC-derived sebocyte precursor cells from human induced pluripotent stem cells (hiPSC).

These hiPSC-derived sebocyte precursor cells have been used to generate a stable and highly homogeneous hiPSC-derived sebocyte population.

Differentiation of hiPSC-derived sebocyte precursor cells into hiPSC-derived sebocytes and maturation of said hiPSC-derived sebocytes may be achieved in the presence of (1) an inhibitor of the SMAD protein signalling pathway, such as the 4-(4-(benzo[d][1,3]dioxol-5-yl)-5-(pyridin-2-yl)-1H-imidazol-2-yl)benzamide (also referred as SB 431542), optionally in combination with an agonist of a peroxisome proliferator activated receptor (PPAR) delta, such as GW0742, and/or (2) a Hedgehog pathway inhibitor, such as cyclopamine.

HiPSC-derived sebocytes generated under these conditions are similar in terms of morphology, marker expression and functions to *in vitro* cultured sebocytes obtained from human individuals.

### • Definitions

Below are given definitions of terms and expressions that are within the scope of the protection that is sought.

The expression "hiPSC-derived sebocyte cells" or the expression "hiPSC-derived sebocytes" is intended to refer to human sebocytes that have been obtained by a differentiation process of hiPSC.

The expression "homogeneous population" is intended to mean that at least 80% of the cells from the cell population express or do not express a given selected marker.

The expression "human induced pluripotent stem cells" or "hiPSC" refers to human adult cells that have been reprogrammed into an embryonic stem cell-like state. hiPSC are therefore capable of expressing genes and factors that are essential for maintaining the defining properties of embryonic stem cells.

The expression "multipotent skin precursor cells" refers to precursor cells that are committed to become skin cells, including, but not limited to, keratinocytes and sebocyte cells.

The expression "hiPSC-derived sebocyte precursor cells" refers to human precursor cells that are committed to become solely sebocytes.

The expression "hiPSC-derived sebocytes" or "human sebocytes" refers to human cells that are genetically programmed to express genes and factors that are essential for maintaining the defining properties of sebocytes.

The expression "basal medium" refers to a medium that comprises all the ingredients that are necessary to grow and expand a defined cell population.

The term "hypo-seborrhea" refers to a decrease in the production of sebum by the sebaceous gland, as compared to the standard average production of sebum by sebaceous gland from a healthy individual.

The term "hyper-seborrhea" refers to an increase in the production of sebum by the sebaceous gland, as compared to the standard average production of sebum by sebaceous gland from a healthy individual.

The term "about" is intended to refer to a range of values that comprise the explicitly given value and further extends to 10% below or above this given value.

### hiPSC-derived sebocyte precursor cells and methods for obtaining the same

One aspect of the invention relates to an *in vitro* method for obtaining a sebocyte precursor cell comprising the steps of:
a) culturing, under feeder layer-free conditions, human induced pluripotent stem cells (hiPSC) in a sebocyte basal medium complemented with a combination of (i) retinoic acid (RA), (ii) bone morphogenetic protein 4 (BMP4) and (iii) epidermal growth factor (EGF), during a period of time ranging from about 5 days to about 15 days, so as to generate multipotent skin precursor cells,
b) culturing, under feeder layer-free conditions, the multipotent skin precursor cells obtained at step a) in a sebocyte differentiation basal medium, during a period of time ranging from about 5 days to about 15 days, so as to generate hiPSC-derived sebocyte precursor cells, and
c) collecting the hiPSC-derived sebocyte precursor cells obtained at step b).

Within the scope of the invention, the expression "under feeder layer-free conditions" is intended to mean that the culture of hiPSC and multipotent skin precursor cells does not require the use of feeder cells, which provide certain nutrients maintaining the cultured cells in an undifferentiated state.

Feeder cells are largely used in culturing various cells of interest, in particular because they are capable of releasing various nutrient compounds in the culture medium, which compounds may benefit to the growth and expansion of the cells of interest. However, the inventors consider that the presence of feeder cells in the culture medium described herein constitute a drawback, because they provide poorly defined and controllable parameters.

Therefore, for the sake of reproducibility of the methods described herein, and in order to avoid lot-to-lot variability, it is important that feeder cells are omitted in the culture system.

In some embodiments, from about 10² cells/cm² to about 10⁵ cells/cm², preferably from about 10³ cells/cm² to about 10⁴ cells/cm², more preferably about 5x10³ cells/cm² of hiPSC are initially inoculated into an appropriate sebocyte basal medium.

hiPSC may be prepared accordingly to Takahashi et al. (Cell; 2007) or any other suitable protocol, in particular a protocol that uses reprogramming factors delivered to somatic cells through, but not limited to, adenoviruses, plasmids, transposons, Sendai viruses, recombinant proteins or synthetic mRNAs (Fontes et al.; 2013; Takahashi and Yamanaka; 2006).

hiPSC may be prepared by reprograming cells by small molecules as described by Hou et al. (Science; 2013).

hiPSC may also be provided commercially, in particular from, but not limited to, ATCC®, THERMOFISHER SCIENTIFIC®, the Coriell Institute, ALSTEM®.

hiPSC may be obtained from individuals belonging to various origin, in particular African individuals, African American individuals, Asian individuals and Caucasian individuals.

In some embodiments, hiPSC may be substituted with any other category of human pluripotent stem cells, in particular human embryonic stem cells (hESC), or any category of human multipotent stem cells, with the proviso that these human stem cells are capable of being differentiated into hiPSC-derived sebocytes.

It is noteworthy to mention that handling human pluripotent stem cells and human multipotent stem cells needs to be performed accordingly to the good practice in ethics.

In some embodiments, an appropriate sebocyte basal medium according to the invention comprises (i) one or more pH buffering system(s); (ii) inorganic salt(s), in particular selected in a group comprising calcium bromide, calcium chloride, calcium phosphate, calcium nitrate, calcium nitrite, calcium sulphate, magnesium bromide, magnesium chloride, magnesium sulphate, potassium bicarbonate, potassium bromide, potassium chloride, potassium dihydrogen phosphate, potassium disulphate, di- potassium hydrogen phosphate, potassium nitrate, potassium nitrite, potassium sulphite, potassium sulphate, sodium bicarbonate, sodium bromide, sodium chloride, sodium disulphate, sodium hydrogen carbonate, sodium dihydrogen phosphate, di-sodium hydrogen phosphate, sodium sulphate and a mix thereof; (iii) trace element(s), in particular selected in a group comprising cobalt (Co), copper (Cu), iron (Fe), magnesium (Mg), manganese (Mn), molybdenum (Mo), nickel (Ni), selenium (Se), zinc (Zn) and the salts thereof; (iv) free amino acid(s), in particular selected in a group comprising L-alanine, L-arginine, L-asparagine, L-aspartic acid, L-cysteine, L-cystine, L-glutamine, L-glutamic acid, glycine, L-histidine, L-isoleucine, L-leucine, L-lysine, L-methionine, L-phenylalanine, L-proline, L-serine, taurine, L-threonine, L-tryptophan, L-tyrosine, L-valine and a mix thereof; (v) vitamin(s), in particular selected in a group comprising biotin (vitamin H); D-calcium-pantothenate; choline chloride; folic acid (vitamin B9); myo-inositol; nicotinamide; pyridoxal (vitamin B6); riboflavin (vitamin B2); thiamine (vitamin B1); cobalamin (vitamin B12); acid ascorbic; α-tocopherol (vitamin E) and a mix thereof; (vi) hormone(s); (vii) carbon/energy source(s), in particular selected in a group comprising D-glucose; pyruvate; lactate; ATP; creatine; creatine phosphate; and a mix thereof

In some preferred embodiments, the basal medium according to the invention is free or substantially free of any compound of non-human animal or human origin.

Within the scope of the invention, a compound of non-human animal or human origin may be selected in a group comprising bovine pituitary extract, human or non-human animal growth factor, bovine serum and foetal bovine serum.

In some embodiments, the basal medium may be complemented with at least one antibiotic selected in a group comprising penicillin, streptomycin, amphotericin B and a mix thereof

In certain embodiments, the sebocyte basal medium may comprise a FAD medium obtained by mixing two commercial media, in particular Dulbecco's modified Eagles' medium (DMEM; THERMOFISHER SCIENTIFIC®) and Ham's F12 medium (THERMOFISHER SCIENTIFIC®), in a respective ratio from about 5:1 (v/v) to about 1:1 (v/v), preferably a ratio of about 3:1 (v/v).

In some embodiments, the sebocyte basal medium may comprise a FAD medium complemented with at least one compound selected in a group comprising foetal bovine serum (FBS), insulin, hydrocortisone, cholera toxin, triiodothyronine, adenine, L-ascorbic acid, and mix thereof.

In some preferred embodiments, the sebocyte basal medium comprises a FAD medium complemented with foetal bovine serum (FBS), insulin, hydrocortisone, cholera toxin, triiodothyronine, adenine and L-ascorbic acid.

When present, FBS may represent from about 0.5% to about 10% w/v of the sebocyte basal medium, preferably from about 1% to about 5%, more preferably about 2%.

When present, insulin may represent a concentration of from about 0.5 µg/ml to about 15 µg/ml in the sebocyte basal medium, preferably of from about 1 µg/ml to about 10 µg/ml, more preferably of about 5 µg/ml.

When present, hydrocortisone may represent a concentration of from about 0.05 µg/ml to about 1.5 µg/ml in the sebocyte basal medium, preferably of from about 0.1 µg/ml to about 1 µg/ml, more preferably of about 0.5 µg/ml.

When present, cholera toxin may represent a concentration of from about 10⁻⁸ M to about 10⁻¹² M in the sebocyte basal medium, preferably of from about 10⁻⁹ M to about 10⁻¹¹ µg/ml, more preferably of about 10⁻¹⁰ M.

When present, triiodothyronine may represent a concentration of from about 0.5 ng/ml to about 5 ng/ml in the sebocyte basal medium, preferably of from about 1 ng/ml to about 2 ng/ml, more preferably of about 1.5 ng/ml.

When present, adenine may represent a concentration of from about 0.5 µg/ml to about 50 µg/ml in the sebocyte basal medium, preferably of from about 5 µg/ml to about 30 µg/ml, more preferably of about 25 µg/ml.

When present, L-ascorbic acid may represent a concentration of from about 0.05 mM to about 15 mM in the sebocyte basal medium, preferably of from about 0.1 mM to about 5 mM, more preferably of about 0.3 mM.

In some embodiments, the sebocyte basal medium may be complemented with from about 0.01 µM to 100 µM of retinoic acid (RA), preferably from about 0.1 µM to 10 µM of RA, more preferably from about 0.5 µM to 5 µM of RA.

In some embodiments, the sebocyte basal medium may be complemented with from about 0.1 ng/ml to about 500 ng/ml of bone morphogenetic protein 4 (BMP4), preferably from about 1 ng/ml to about 100 ng/ml of BMP4, more preferably from about 10 ng/ml to about 50 ng/ml of BMP4.

In some embodiments, sebocyte basal medium may be complemented with from about 0.1 ng/ml to about 500 ng/ml of epidermal growth factor (EGF), preferably from about 1 ng/ml to about 100 ng/ml of EGF, more preferably from about 10 ng/ml to about 50 ng/ml of EGF.

In some preferred embodiments, the sebocyte basal medium is complemented with:
- from about 0.01 µM to 100 µM of retinoic acid (RA), preferably from about 0.1 µM to 10 µM of RA, more preferably from about 0.5 µM to 5 µM of RA;
- from about 0.1 ng/ml to about 500 ng/ml of bone morphogenetic protein 4 (BMP4), preferably from about 1 ng/ml to about 100 ng/ml of BMP4, more preferably from about 10 ng/ml to about 50 ng/ml of BMP4; and
- from about 0.1 ng/ml to about 500 ng/ml of epidermal growth factor (EGF), preferably from about 1 ng/ml to about 100 ng/ml of EGF, more preferably from about 10 ng/ml to about 50 ng/ml of EGF.

In practice, the culturing at step a) is performed at a temperature of from about 32°C to about 42°C, preferably of from about 35°C to about 39°C, more preferably of about 37°C.

In practice, the culturing at step a) is performed with a CO₂ concentration of from about 1% to 10%, preferably of from about 2.5% to about 7.5%, more preferably of about 5%.

In some embodiments, the sebocyte basal medium of step a) is removed once or twice during the time course of step a) and replaced with a fresh medium.

In some particular embodiments, the culturing of hiPSC may be performed in step a) in the form of a monolayer.

The inventors have surprisingly observed that culturing the hiPSC as a monolayer may significantly improve the homogeneity of the hiPSC-derived sebocyte precursor cells within the cell population, in particular with respect to the percentage of cells expressing a given marker within the cell population at the end of step c).

Furthermore, the inventors have also surprisingly observed that culturing the hiPSC as a monolayer may significantly influence the total count of hiPSC-derived sebocyte precursor cells within the cell population, since more hiPSC-derived sebocyte precursor cells may be obtained from hiPSC at the end of step c).

Without wishing to be bound by a theory, the inventors believe that culturing the hiPSC accordingly to these particular embodiments, i.e. in the form of a monolayer, is very suitable for generating hiPSC-derived sebocyte precursor cells in large scale.

Within the scope of the invention, the expression "fresh medium" is intended to mean a medium that has not been in contact with any type of cells.

In certain embodiments, an appropriate differentiation basal medium for performing step b) may be obtained by mixing 2 commercial media, such as Defined keratinocyte SFM medium (THERMOFISHER SCIENTIFIC®) and keratinocyte-SFM medium (THERMOFISHER SCIENTIFIC®), in particular in a ratio of respectively from about 2:1 to about 1:2, more preferably in a ratio of respectively about 1:1.

In some embodiments, the differentiation basal medium of step b) is removed every day or every other day during the time course of step b) and replaced with a fresh medium.

In certain embodiments, the culturing in step a), step b) or step a) and step b) is performed during a period of time ranging from about 9 days to about 13 days, more preferably of about 11 days.

In some embodiments, the *in vitro* method further comprises a step d) of freezing the hiPSC-derived sebocyte precursor cells collected at step c).

In practice, the hiPSC-derived sebocyte precursor cells may be frozen, in particular in liquid nitrogen, and subsequently maintained at a temperature comprised between about -20°C to about -150°C.

The inventors surprisingly observed that the step d) of freezing the hiPSC-derived sebocyte precursor cells, also referred as to a cryogenization step, may significantly increase the homogeneity of cells within the cell population after thawing and, in particular, may increase the percentage of cells expressing one or more marker(s) of interest within the cell population.

Another aspect of the invention relates to hiPSC-derived sebocyte precursor cells obtained by the *in vitro* method as described herein.

Another aspect of the invention relates to hiPSC-derived sebocyte precursor cell population obtained by the *in vitro* method as described herein.

In some embodiments, hiPSC-derived sebocyte precursor cells are KRT7+/MUC1+ hiPSC-derived sebocyte precursor cells.

Within the scope of the invention, the expression "KRT7+ cell" refers to a cell expressing the KRT7 gene that encodes the KRT7 protein.

In practice, KRT7 may be equivalent to any one of the aliases, such as keratin 7, sarcolectin, type II keratin kb7, cytokeratin 7, CK7, SCL, K7, K2C7.

Within the scope of the invention, the term "MUC1+ cell" refers to a cell expressing the MUC1 gene that encodes the cell surface associated mucin 1 protein (MUC1).

In practice, MUC1 may be equivalent to any one of the aliases, such as Tumor-Associated Epithelial Membrane Antigen, Breast Carcinoma-Associated Antigen DF3, Peanut-Reactive Urinary Mucin, Polymorphic Epithelial Mucin, Carcinoma-Associated Mucin, Mucin 1, Krebs Von Den Lungen-6, Cancer Antigen 15-3, Episialin, CA 15-3, H23AG, KL-6, PEMT, EMA, PUM, PEM, Medullary Cystic Kidney Disease (1), Tumor Associated Epithelial Mucin, Tumor-Associated Mucin, CD227 Antigen, DF3 Antigen, H23 Antigen, MUC-1/SEC, ADMCKD1, MUC-1/X, MUC1/ZD, ADMCKD, CD227, MCKD1, MAM6, MCKD and MCD.

It is the responsibility of a person skilled in the art to assess the expression of the markers accordingly to the suitable methods in the art.

In some embodiments, the detection and/or measurement of the expression of KRT7 and/or MUC1 markers may be performed at the mRNA level, in particular by the mean of suitable nucleic acid probes.

In some embodiments, the expression of markers of interest is measured by qPCR.

In some embodiments, the detection and/or measurement of the expression of KRT7 and/or MUC1 markers may be performed at the protein level, in particular by the mean of specific ligand directed against these proteins, such as pluriclonal or monoclonal antibodies, aptamers and the like.

In some embodiments, the hiPSC-derived sebocyte precursor cell population comprises more than 80% of cells expressing the KRT7 marker.

Within the scope of the instant invention, the expression more than 80% includes 81%, 82%, 83%, 84%, 85%, 86%, 87%, 88%, 89%, 90%, 91%, 92%, 93%, 94%, 95%, 96%, 97%, 98%, 99% and 100%.

In certain embodiments, the hiPSC-derived sebocyte precursor cell population comprises more than 90% of cells expressing KRT7.

In some embodiments, the hiPSC-derived sebocyte precursor cell population comprises from about 20% to about 40% of cells expressing the MUC1 marker.

Within the scope of the instant invention, the expression "from about 20% to about 40%" includes 21%, 22%, 23%, 24%, 25%, 26%, 27%, 28%, 29%, 30%, 31%, 32%, 33%, 34%, 35%, 36%, 37%, 38% and 39%.

### • hiPSC-derived sebocytes and methods for obtaining the same

Another aspect of the invention relates to an *in vitro* method for obtaining hiPSC-derived sebocytes comprising step a) of culturing, under feeder layer-free conditions, the hiPSC-derived sebocyte precursor cells obtained by the method as described above, in a sebocyte basal medium complemented by an inhibitor of the Small Mothers Against Decapentaplegic (SMAD) protein signalling pathway, during a period of time of about 2 days to about 7 days.

In some embodiment, the culturing of step a) is performed during a period of about 3 days.

The SMAD protein signalling pathway, also referred as to the TGF-β/SMAD signalling pathway, plays a critical role in the regulation of homeostasis, in particular by controlling cellular proliferation, survival, differentiation and migration, as well as in various developmental processes. Upon binding of specific ligands to cognate cell surface receptors, SMAD proteins become activated and translocate to the nucleus where they partner with transcription factors and modulate gene expression.

In some embodiments, the inhibitor of the SMAD protein signalling pathway is selected in a group comprising 4-(4-(benzo[d][1,3]dioxol-5-yl)-5-(pyridin-2-yl)-1H-imidazol-2-yl)benzamide (SB431542); 4-(2-(6-methylpyridin-2-yl)-5,6-dihydro-4H-pyrrolo[1,2-b]pyrazol-3-yl)quinoline-6-carboxamide (Galunisertib or LY2157299); 7-(2-morpholinoethoxy)-4-(2-(pyridin-2-yl)-5,6-dihydro-4H-pyrrolo[1,2-b]pyrazol-3-yl)quinoline (LY2109761); 6-(2-tert-butyl-4-(6-methylpyridin-2-yl)-1H-imidazol-5-yl)quinoxaline (SB525334); 2-(4-(benzo[d][1,3]dioxol-5-yl)-2-tert-butyl-1H-imidazol-5-yl)-6-methylpyridine (SB505124); 4-(4-(3-(pyridin-2-yl)-1H-pyrazol-4-yl)pyridin-2-yl)-N-(tetrahydro-2H-pyran-4-yl)benzamide (GW788388); 4-[3-(2-pyridinyl)-1H-pyrazol-4-yl]-quinoline (LY364947); 2-[3-(6-methyl-2-pyridinyl)-1H-pyrazol-4-yl]-1,5-Naphthyridine (RepSox); 3-[(6-Amino-5-(3,4,5-trimethoxyphenyl)-3-pyridinyl]phenol (K02288); 2-(5-chloro-2-fluorophenyl)-N-(pyridin-4-yl)pteridin-4-amine (SD-208); N-(2-fluorophenyl)-5-(6-methyl-2-pyridinyl)-4-[1,2,4]triazolo[1,5-a]pyridin-6-yl-1H-imidazole-2-methanamine (EW-7197); 5-[6-[4-(1-piperazinyl)phenyl]pyrazolo[1,5-a]pyrimidin-3-yl]-quinoline (LDN-212854); and 2-[([1,1'-biphenyl]-4-ylamino)carbonyl]-benzoic acid (Kartogenin).

In practice, the inhibitors of the SMAD protein signalling pathway mentioned above may be commercially available from TOCRIS® and SELLECKCHEM®.

In certain embodiments, the said inhibitor of the SMAD protein signalling pathway is 4-(4-(benzo[d][1,3]dioxol-5-yl)-5-(pyridin-2-yl)-1H-imidazol-2-yl)benzamide (SB 431542).

It is known in the art that the SMAD protein signalling pathway involves several TGF-beta receptors, such as ALK4, ALK5 and ALK7.

TGF-beta receptor (ALK5) belongs to the SMAD protein signalling pathway. Activation decreases sebocyte differentiation in *in vitro* cultured sebaceous gland (Downie et al., 2002), and in *in vitro* cultured primary sebocytes (McNairn et al., 2013). On the other hand, TGF-beta receptor inhibition by shRNA increases lipogenesis in *in vitro* cultured primary sebocytes (McNairn et al., 2013).

In some embodiment, the said sebocyte basal medium is further complemented by an agonist of peroxisome proliferator activated receptor (PPAR) delta in the said hiPSC-derived sebocytes.

In certain embodiments, the agonist of peroxisome proliferator activated receptor (PPAR) delta is GW0742.

GW0742 has been previously shown to promote lipogenesis in rat preputial sebocytes *in vitro* (Deplewski et al. 2011) and immortalized SZ95 sebocytes (Trivedi et al. 2006).

Surprisingly, the inventors noticed that the GW0742, as used herein, does not affect lipogenesis induction, when in combination with SB 431542. However, the inventors observed that GW0742 improves hiPSC-derived sebocyte cells sensitivity to testosterone or DHT. Therefore, a combination of SB 431542 and GW0742 results in a significantly improved lipogenesis upon stimulation of sebocytes with testosterone or DHT.

Without wishing to be linked to a theory, the inventors believe that GW0742 may stimulate the expression of the androgen receptor. It is known that upon testosterone or DHT stimulation, these compounds interact with nuclear androgen receptors. Once activated, androgen receptors translocate into the nucleus and initiate their effects via genomic signalling.

For the first time, starting from hiPSC, the inventors were able to generate hiPSC-derived sebocytes that can be responsive to testosterone or DHT stimulation, and hence mimic physiological situations, as it was shown *in vivo* that testosterone stimulation increases lipid production in the sebaceous gland (Makrantonaki, 2011).

In some embodiment, the *in vitro* method as described herein, further comprises the step b) of culturing, under feeder layer-free conditions, the hiPSC-derived sebocytes obtained at step a) in a sebocyte basal medium complemented by a compound capable of inducing lipogenesis in the said hiPSC-derived sebocytes, during a period of time of about 2 days to about 7 days.

In some embodiment, the culturing of step b) is performed during a period of about 4 days.

In some embodiment, the compound capable of inducing lipogenesis is selected in a group comprising a Hedgehog pathway inhibitor.

In certain embodiments, the Hedgehog pathway inhibitor is selected in a group comprising cyclopamine, KAAD-cyclopamine, Jervine, Staurosporinone, Physalin B, Physalin F, Sant 1, Sant 2, Sant 3, Sant 4, Cur-61414, GANT58, GANT61, Robotnikinin, IPI-926 (Saridegib), R51,211 (Itraconazole), and GDC-0449 (Vismodegib).

Cyclopamine has been previously shown as promoting increased differentiation of sebocytes, as evidenced by increased lipid accumulation in immortalized sebocytes SZ-95 (Niemann et al., 2003).

As shown in the example section hereunder, hiPSC-derived sebocytes that are cultured sequentially in the presence of (1) SB 431542 or a mixture of SB 431542 and GW0742 and (2) cyclopamine, present an increase ability to respond to LA, testosterone or DHT stimulation, resulting in an increased induction of lipogenesis.

Hence, sebocytes that are cultured accordingly to the methods described herein provide hiPSC-derived sebocytes adapted for assessing naturally occurring physiological phenomena, such as sebum production under various conditions, or pathological conditions, such as hypo-seborrhea or hyper-seborrhea.

Another aspect of the invention relates to hiPSC-derived sebocytes obtained by an *in vitro* method as described herein.

Another aspect of the invention relates to a hiPSC-derived sebocyte population obtained by an *in vitro* method as described herein.

In some embodiments, the hiPSC-derived sebocyte cell population comprises more than 80% of cells expressing the KRT7 marker and/or the MUC1 marker.

In some embodiments, the hiPSC-derived sebocyte cell population comprises more than 80% of cells expressing the KRT7 and MUC1 markers.

Within the scope of the instant invention, the expression more than 80% includes 81%, 82%, 83%, 84%, 85%, 86%, 87%, 88%, 89%, 90%, 91%, 92%, 93%, 94%, 95%, 96%, 97%, 98%, 99% and 100%.

In certain embodiments, the hiPSC-derived sebocyte cell population comprises more than 90% of cells expressing the KRT7 marker and/or the MUC1 marker.

In some embodiments, the hiPSC-derived sebocyte cell population comprises more than 90% of cells expressing the KRT7 and MUC1 markers.

### • Uses of hiPSC-derived sebocytes and methods for using the same

Uses and methods described hereunder are implemented *ex vivo* or *in vitro*, preferably *in vitro*.

One aspect of the invention relates to the use of hiPSC-derived sebocyte precursor cells and/or hiPSC-derived sebocytes as described herein, for the preparation of an *in vitro* human model of hypo-seborrheic human skin or hyper-seborrheic human skin.

In some other aspect, the invention also relates to a method for the preparation of an *in vitro* human model of hypo-seborrheic human skin or hyper-seborrheic human skin comprising the step of providing hiPSC-derived sebocyte precursor cells and/or hiPSC-derived sebocytes as described herein.

Another aspect of the invention relates to the use of hiPSC-derived sebocyte precursor cells and/or hiPSC-derived sebocytes as described herein, for the preparation of an *in vitro* human model of 3D skin epithelium.

The invention also relates to a method for the preparation of an *in vitro* human model of 3D skin epithelium comprising the step of providing hiPSC-derived sebocyte precursor cells and/or hiPSC-derived sebocytes as described herein.

In some embodiments, for the purpose of providing an *in vitro* human model of skin, the hiPSC-derived sebocytes may be mixed with other cell types, which are found in the skin, in particular keratinocytes, melanocytes, fibroblasts, Langerhans cells and the like.

In some embodiments, for the purpose of providing *in vitro* human model of skin, the hiPSC-derived sebocytes may be mixed with collagen, elastin, extracellular matrix and a mixture thereof.

In a still other aspect, the invention relates to the use of hiPSC-derived sebocytes as described herein, for the preparation of an *in vitro* human model of testosterone-sensitive cells.

The invention also relates to a method for the preparation of an *in vitro* human model of testosterone-sensitive cells comprising the step of providing hiPSC-derived sebocytes as described herein.

In some embodiments, the *in vitro* human model of testosterone-sensitive cells may be used for assessing the hiPSC-derived sebocytes response upon stimulation with testosterone or dihydrotestosterone.

In a further aspect, the invention also relates to the use of hiPSC-derived sebocytes as described herein, for the preparation of an *in vitro* human model of linoleic acid-sensitive cells.

The invention also relates to a method for the preparation of an *in vitro* human model of linoleic acid-sensitive cells comprising the step of providing hiPSC-derived sebocytes as described herein.

In a further aspect, the invention also relates to the use of hiPSC-derived sebocytes as described herein, as a tool for screening a compound suitable for the treatment and/or the prevention of the dysfunction of a sebaceous gland.

The invention also relates to a method for screening a compound suitable for the treatment and/or the prevention of the dysfunction of a sebaceous gland comprising the steps of:
- (i) providing hiPSC-derived sebocytes as described herein;
- (ii) contacting the cells of step (i) with a test compound;
- (iii) measuring at least one parameter associated with a dysfunction of a sebaceous gland;
- (iv) comparing said at least one parameter with a reference value;
wherein the detection of a variation between the parameter measured in step (iii) and the reference value indicate the capacity of the test compound to treat and/or prevent the said dysfunction of a sebaceous gland.

In certain embodiments, the parameter is selected in a group comprising the expression of at least one marker, in particular a gene or a protein, associated with lipogenesis and the lipid total content of the human sebocyte cells.

In some embodiments, the at least one marker is selected in a group comprising, PPARG, FADS2, SCD, AWAT1, PLIN2 and FDFT1.

Within the scope of the present description, the expression "at least one" includes one, two, three, four, five, six, seven, eight, nine, ten or more.

In some embodiments, the reference value may be measured in sebocytes in the absence of a compound known to affect the function of the sebaceous glands.

In some embodiments, a dysfunction of a sebaceous gland may be selected in a group comprising, but not limited to, hypo-seborrhea, hyper-seborrhea, acne vulgaris, rosacea, rosacea-like dermatitis, lupus miliaris disseminatus faciei, atopic dermatitis, seborrheic dermatitis and xerosis.

In another aspect, the invention also relates to the use of hiPSC-derived sebocytes as described herein, as a tool for screening a compound suitable for the treatment and/or the prevention of hypo-seborrheic or hyper-seborrheic conditions of the human skin.

The invention also relates to a method for screening a compound suitable for the treatment and/or the prevention of hypo-seborrheic or hyper-seborrheic conditions of the human skin comprising the steps of:
- (i) providing hiPSC-derived sebocytes as described herein;
- (ii) contacting the cells of step (i) with a test compound;
- (iii) measuring at least one parameter associated with a hypo-seborrheic or a hyper-seborrheic condition of the human skin;
- (iv) comparing said at least one parameter with a reference value;
wherein the detection of a variation between the parameter measured in step (iii) and the reference value indicates the capacity of the test compound to treat and/or prevent a hypo-seborrheic or a hyper-seborrheic condition of the human skin.

In practice, when the parameter is the total lipid content of sebocytes, a variation of the measured total lipid content in the presence of a test compound as compared to the total lipid content of sebocytes measured in the absence of any test compound is indicative of the ability of said test compound to treat and/or prevent a hypo-seborrheic or a hyper-seborrheic condition of the human skin.

For example, an increase of the total lipid content in the presence of a test compound as compared to the total lipid content of sebocytes measured in the absence of any test compound is indicative of the ability of said test compound to treat and/or prevent a hypo-seborrheic condition of the human skin.

Similarly, a decrease of the total lipid content in the presence of a test compound as compared to the total lipid content of sebocytes measured in the absence of any test compound is indicative of the ability of said test compound to treat and/or prevent a hyper-seborrheic condition of the human skin.

### • Characterisation of the hiPSC-derived sebocyte precursor cells and

### the hiPSC-derived sebocytes obtained by the methods described herein

In some embodiments, the cell populations described herein may be characterised by the mean of a phenotypic analysis.

In some embodiments, the phenotypic analysis may be performed by assessing the expression of at least one marker, in particular a marker selected in a group comprising KRT7, MUC1, PPARG, FADS2, SCD, MC5R, AWAT1, PLIN2, FDFT1, Androgen Receptor (AR).

In practice, PPARG may be equivalent to any one of the aliases, such as Peroxisome Proliferator Activated Receptor Gamma, Nuclear Receptor Subfamily 1 Group C Member 3, PPAR-Gamma, NR1C3, Peroxisome Proliferator-Activated Nuclear Receptor Gamma Variant 1, PPARgamma, PPARG1, PPARG2, CIMT1 and GLM1.

In practice, FADS2 may be equivalent to any one of the aliases, such as Fatty Acid Desaturase 2, Delta(6) Fatty Acid Desaturase, Acyl-CoA 6-Desaturase, Delta 6 Desaturase, D6D, Linoleoyl-CoA Desaturase (Delta-6-Desaturase)-Like 2, Delta-6 Fatty Acid Desaturase, EC 1.14.19.3, EC 1.14.19 6, SLL0262, FADSD6, LLCDL2, DES6 and TU13.

In practice, SCD may be equivalent to any one of the aliases, such as Stearoyl-CoA Desaturase (Delta-9-Desaturase), Fatty Acid Desaturase, Stearoyl-CoA Desaturase Opposite Strand, Delta-9 Desaturase, EC 1.14.19.1, HSCD1, Stearoyl-CoA Desaturase, Acyl-CoA Desaturase, MSTP008, FADS5, SCDOS, SCD1.

In practice, MC5R may be equivalent to any one of the aliases, such as Melanocortin 5 Receptor, MC5-R, MC-2 and MC2.

In practice, AWAT1 may be equivalent to any one of the aliases, such as Acyl-CoA Wax Alcohol Acyltransferase 1, Diacylglycerol O-Acyltransferase 2-Like Protein 3, Long-Chain-Alcohol O-Fatty-Acyltransferase 1, Diacylglycerol O-Acyltransferase 2-Like 3, Diacylglycerol Acyltransferase 2, DGAT2L3, DGA2, and EC 2.3.1.75.

In practice, PLIN2 may be equivalent to any one of the aliases, such as Perilipin 2, Adipose Differentiation-Related Protein, Adipophilin, ADFP and ADRP.

In practice, FDFT1 may be equivalent to any one of the aliases, Famesyl-Diphosphate Farnesyltransferase 1, FPP:FPP Farnesyltransferase, EC 2.5.1.21, SQS, SS, Farnesyl-Diphosphate Farnesyltransferase, Presqualene-Di-Diphosphate Synthase, Squalene Synthetase, Squalene Synthase, DGPT and ERG9.

In practice, AR may be equivalent to any one of the aliases, KD, AIS, AR8, TFM, DHTR, SBMA, HYSP1, NR3C4, SMAX1, HUMARA.

In some embodiments, the cell lines described herein may be characterised by their ability to achieve lipogenesis, upon linoleic acid (LA), testosterone or dihydrotestosterone induction.

Lipogenesis may be evaluated by performing any suitable method known in the art.

For example, the capacity of hiPSC-derived sebocytes to achieve lipogenesis may be qualitatively and/or quantitatively evaluated by the use of fluorescent probes that are specific of lipids, in particular BODIPY fluorophores, and further fluorescence detection with appropriate materials.

In some other embodiments, the total lipid content of the hiPSC-derived sebocytes obtained by a method described herein may be evaluated, by performing any suitable method known in the art.

For example, the total lipid content profile of the hiPSC-derived sebocytes may be obtained by Gaz Chromatography/Mass Spectometry (GC/MS).

### EXAMPLES

### Example 1 - Production of hiPSC-derived sebocyte precursor cells

### 1- Material

hiPSC were qualified under the standards provided by the International Society for Stem Cell Research (ISSCR) and accordingly to Soares et al. (Stem Cell Rep. 2014).

Culture media, such as StemMACS® iPS-Brew XF was obtained from MILTENYI BIOTEC®.

FAD medium is obtained by a mixture of the following ingredients (for 100 mL):
- 3 vol. of DMEM-Glutamax + 1 vol. of Ham F12: 92 mL
- 2% FBS: 2 mL
- 5 µg/mL insulin: 50 µL
- 0.5 µg/mL hydrocortisone : 1 mL
- 10⁻¹⁰ M cholera toxin : 0.9 µL
- 1.37 ng/mL triiodothyronine: 6.7 µL
- 24 µg/mL adenine: 4.8 mL
- 0.3 mM L-ascorbic acid: 115.7 µL

FAD culture medium is complemented as detailed in the table below.

| | |
|---|---|
| Retinoic acid (RA) | dl-d10: 1 µM |
| Bone morphogenetic protein 4 (BMP4) | d3-d10: 25 ng/mL |
| Epidermal growth factor (EGF) | d3-d10: 20 ng/mL |

Defined keratinocytes SFM (DK-SFM) and Keratinocyte SFM (K-SFM) commercial media are from THERMOFISHER SCIENTIFIC®.

TrypLE™ Express is a commercial enzymatic solution from THERMOFISHER SCIENTIFIC®.

### 2- Method

Figure 2 is illustrating the overall culturing strategy.

### a) hiPSC seeding (d-5 to d0)

When hiPSC would normally be ready to passage, aspirate the media and add 600 µL of accutase solution per 6-well plate.

Incubate the cell culture plate at 37°c for 10 to 15 min and harvest the colonies in StemMACS™ iPS-Brew XF medium.
- Triturate cells in cell culture plate.
- Centrifuge cells for 3 minutes at 200xg at room temperature.
- Determine the cell concentration using a hemacytometer.
- Plate cells in 3 wells of a 6-wells cell culture plate at 5 000 cells/cm².
- After 5 days, when cells are 90% confluent, differentiation is initiated.

### b) Differentiation

Day 0 : Replace the StemMACS™ iPS-Brew XF medium with FAD medium containing 1 µM retinoic acid.
Day 3 to Day 10: Change medium every other days with FAD medium complemented with 1 µM retinoic acid, 20 ng/mL EGF and 25 ng/mL BMP4.
Day 10 to 22: Change medium with DKSFM/KSFM (1 vol:1 vol) containing 20 ng/mL EGF and 1 ng/mL BMP4.

### c) Freezing of the sebocvte precursor cells

At Day 22, treat cells with collagenase IV for 4 hours at 37°C.

Dilute collagenase IV with DKSFM/KSFM (1 vol:1 vol) and spin at 250xg for 3 min.

Discard supernatant and add 1mL of TrypLE. Incubate at 37°C for 15 min.

Dilute TrypLE™ Express with DKSFM/KSFM (1 vol:1 vol) and spin at 250xg for 3 min.

Resuspend cells at 2x10⁶ cells per mL in CS10 and freeze immediately at - 80°C in a progressive freezing container.

### 3- Characterization of the hiPSC-derived sebocyte precursor cells

Purity of hiPSC-derived sebocyte precursor cells was assessed by flow cytometry (Figure 3). More than 90% of the cells express the KRT7 marker (Figure 3, plot 2).

The expression of specific markers, such as KRT7 (Figure 4), MUC1 (Figure 5) and PPARG (Figure 6) is highly up-regulated in hiPSC-derived sebocytes when compared to the expression of these markers in hiPSC or primary keratinocytes.

### Example 2 - Linoleic acid response of hiPSC-derived sebocytes cultured in FAD medium (basal medium) supplemented with SB431542 ("supplement A")

The hiPSC-derived sebocyte precursor cells obtained as in example 1 above were culture in basal medium (Sebocyte basal medium, see below) complemented with 10 µM final of TGF-beta inhibitor consisting in 4-[4-(1,3-benzodioxol-5-yl)-5-(2-pyridinyl)-1H-imidazol-2-yl]benzamide, also designated "SB431542" (TOCRIS®) ("supplement A"), as disclosed in Figure 7.

All steps should be performed in a sterile culture environment using adequate handling procedures. hiPSC-derived sebocytes are human cells and, as such, should be handled with required ethical and safety rules.

### 1- Culture of hiPSC-derived sebocytes

### a) Reagents and media:

▪ Sebocyte basal medium (filter sterilize and store at 4°C for up to a month):
   ∘ 3:1 (vol:vol) DMEM-glutamax (THERMOFISCHER SCIENTIFIC®) :
      Ham's F12 (THERMOFISCHER SCIENTIFIC®)
   ∘ 2.5% FBS (SIGMA-ALDRICH®)
   ∘ 10 ng/mL insulin (SIGMA-ALDRICH®)
   ∘ 3 ng/mL EGF (THERMOFISCHER SCIENTIFIC®)
   ∘ 45.2 ng/mL hydrocortisone (SIGMA-ALDRICH®)
   ∘ 10⁻¹⁰M cholera toxin (SIGMA-ALDRICH®)
   ∘ 24 µg/mL adenine (MERCK MILLIPORE®)
▪ Supplement A (stored at -20°C)
▪ Fibronectin (SIGMA-ALDRICH®)
▪ Sebocyte complete medium: extratemporaneously add 1:2000 (vol:vol) supplement A to adequate volume of sebocyte basal medium.

### b) Procedure:

1. At d0, coat tissue culture plates with fibronectin diluted to 1/100 in PBS. Incubate for at least 2 h in 37°C incubator (use 0.1 mL per cm² cell culture surface).
2. Pre-warm sebocyte basal medium.
3. Quickly thaw one hiPSC-derived sebocytes vial in a 37°C water bath.
4. Wipe out the outside of the vial with 70% ethanol.
5. Transfer the cells to 6 mL of sebocyte basal medium.
6. Centrifuge at 250xg for 3 min, discard supernatant and resuspend in 2 mL of sebocyte basal medium.
7. Count cells; remove fibronectin solution from the culture plates and directly plate cells on fibronectin-coated surface at a density of 25,000 cells/cm². Use 2 mL sebocyte basal medium per 10 cm² of culture surface.
8. Place the plate into the incubator (37°C, 5% CO₂). Evenly distribute the cells.
9. Replace medium next day (d1, the day after thawing) and every other day thereafter for 2 or 3 days with the sebocyte basal medium complemented with or without supplement A.

### 2- Response to linoleic acid

Linoleic acid (LA) response from hiPSC-derived sebocytes cultured for 3 days as described above, was assessed following the protocol detailed hereunder.

hiPSC-derived sebocytes are fixed with 4% Paraformaldehyde (SIGMA-ALDRISCH®) and stained with Bodipy (THERMOFISHER SCIENTIFIC®) according to the manufacturer instructions. Imaging and quantification of fluorescence were performed on a CX5 automated imager (THERMOFISHER SCIENTIFIC®).

Figure 8 shows that lipogenesis induced by the LA response from sebocytes at d3 is correlated with the presence of SB431542 in the basal medium.

After a 24 h treatment of hiPSC-derived sebocytes with increasing amounts of LA, sebocytes display a dose-dependent response to LA induction (Figure 9).

Therefore, a treatment of sebocytes with SB431542 induces a strong response to LA, resulting in the activation of lipogenesis pathway that correlates with the induction of the lipogenesis pathway.

### Example 3 - Dihydrotestosterone (DHT) response of hiPSC-derived sebocytes cultured in basal medium supplemented with SB431542 ("supplement A") or with SB431542 and GW0742 ("supplement A+C")

Figure 10 illustrates the culture parameters. hiPSC-derived sebocyte precursor cells are thawed and cultured for 5 days in conditions similar to example 2.

"Supplement A+C" represents a mixture of "supplement A" (SB431542; final concentration of 10 µM) with "supplement C" (GW0742; final concentration of 1 µM).

As shown in Figure 11, lipogenesis induction in response to DHT in sebocytes is only correlated with the presence of SB431542 and GW0742 in the basal medium.

### Example 4 - Linoleic response of hiPSC-derived sebocytes cultured in FAD medium (basal medium) supplemented with cyclopamine ("supplement B")

The hiPSC-derived sebocyte precursor cells obtained as in example 1 above were culture in basal medium (FAD medium of example 1) complemented with 5 µM final of the Hedgehog signaling inhibitor cyclopamine (TOCRIS®) ("supplement B"), as disclosed in Figure 12.

### 1- Culture of the sebocytes

### a) Reagents and media

▪ Sebocyte basal medium (see example 2 above).
▪ Filter sterilize and store at 4°C.
▪ Supplement B stored at -20°C.
▪ Fibronectin (SIGMA-ALDRICH®).
▪ Sebocyte complete medium: extratemporaneously add 1:1000 (vol:vol) supplement B to adequate volume of sebocyte basal medium.

### b) Procedures

### b1) From d0 to d5

1-8. Steps are performed as in example 2 above.
9. Replace medium next day (d1, the day after thawing) and every other day thereafter for 4 days with the sebocyte basal medium complemented with supplement B.

### b2) Passaging of hiPSC-derived sebocytes at d5

Reagents:
▪ Sebocyte complete medium
▪ TrypLE™ Express (THERMO FISCHER®)
▪ Fibronectin (SIGMA-ALDRICH®)

Protocol:
1. For hiPSC-derived sebocytes maintenance, passage is usually performed when cells reach 90% confluence (usually within 5-6 days after thawing). One can expect a yield of about 50,000 cells/cm².
2. Coat tissue culture plate with fibronectin diluted to 1/100 in PBS. Incubate for at least 2 h in 37°C incubator. Before use, remove fibronectin solution.
3. Pre-warm sebocyte complete medium and TrypLE™ Express.
4. Discard culture medium, briefly wash once with PBS.
5. Add 1 mL TrypLE™ Express per 10 cm² of culture surface, and incubate at 37°C for 5-10 min. Regularly check cell digestion: when sebocytes are rounding up, detach them by gently flushing the culture surface with the TrypLE™ Express contained in the plate.
6. Transfer to a 15 mL tube pre-loaded with sebocyte complete medium (at least a 1/3 dilution ratio is necessary to stop TrypLE™ Express action).
7. Centrifuge at RT, 250xg for 3 min.
8. Eliminate supernatant and re-suspend in sebocyte complete medium. Gently triturate until a single cell solution is achieved.
9. Count cells and plate on fibronectin coated culture surface at a density of 25,000 cells/cm².
10. Place the plate into the incubator. Evenly distribute the cells.
11. Replace medium every other day.

### 2- Response to linoleic acid induction of the sebocytes from d5

Linoleic acid (LA) response from sebocytes cultured for 5 days as described above, was assessed as disclosed in example 2.

After a 24 h treatment of sebocytes with varying concentrations of linoleic acid (LA), sebocytes display a dose-dependent response to linoleic acid induction (Figure 13).

### Example 5 - Characterization of hiPSC-derived sebocytes cultured sequentially for 3 days in basal medium supplemented with SB431542 and GW0742 ("supplement A+C") and for 2 extra days in basal medium supplemented with cyclopamine ("supplement B")

Figure 14 illustrates the culture parameters.

### 1- Culture of the sebocytes

### a) Reagents and media

Sebocyte basal medium is as described in example 1.

Supplement C is as in example 3.

### b) Procedure

1. Coat tissue culture plates with fibronectin diluted to 1/100 in PBS. Incubate for at least 2 h in 37°C incubator.
2. Pre-warm sebocyte complete medium.
3. Quickly thaw hiPSC-derived sebocytes vial in 37°C water bath until a small frozen cell pellet remains. Do not vortex cells.
4. Wipe out the outside of the vial of cells with 70% ethanol.
5. Transfer the cells to 6 mL of sebocyte basal medium.
6. Centrifuge at 250xg for 3 min, discard supernatant and resuspend in 2 mL of sebocyte basal medium supplemented with 1:1000 supplement A+C.
7. Count cells; remove fibronectin solution from the culture plates and directly plate on fibronectin-coated surface at a density of 25,000 cells/cm². Use 2 mL sebocyte medium with 1:1000 supplement A+C per 10 cm² of culture surface.
8. Place the plate into the incubator (37°C, 5% CO₂).
9. Use sebocyte basal medium supplemented with 1:1000 supplement A+C for at least 3 days after plating, then switch to sebocyte basal medium supplemented with 1:1000 supplement B for the next 2 days until cells reach 90% confluence and can be further passaged. Change medium every other day using 2 mL/10 cm² culture surface.

### 2- Phenotypic characterization of the hiPSC-derived sebocytes at d5

Phenotypic characterization of the sebocytes at d5 is performed as in example 1 and is presented in Figures 15, 16, 17 and 18.

Similarly to the sebocyte precursor cells obtained as described in example 1, the mature sebocytes strongly express KRT7 and MUC1 markers (respectively Figures 15 and 16), as compared to iPSC and primary keratinocytes.

In addition, functional markers such as lipid activated transcription factor PPARG (Figure 17) and enzymes of the fatty acid biosynthesis pathway, in particular FADS2, AWAT1 (wax esters), FDFT1 (squalene synthase) are also strongly expressed (Figure 18) in sebocytes.

It is to be noted that FDFT1 is also expressed in keratinocytes (Figure 18), as described in Pappas (2009).

Additional functional markers expressed in sebocytes also include the lipid droplet associated protein PLIN2 (Figure 18).

### Example 6 - LA response of hiPSC-derived sebocytes cultured for 5 days in basal medium complemented with SB431542 and GW0742 ("supplement A+C") or cultured sequentially for 3 days in basal medium complemented with supplement A+C and for 2 days in basal medium complemented with cyclopamine ("supplement B")

Figure 19 illustrates the culture parameters. Sebocyte cells are cultured for 3 days in basal medium in the presence of "supplement A+C" and for 2 additional days in either "supplement A+C" or "supplement B" ("supp A+C+B"). Lipogenesis is assessed upon LA stimulation of sebocytes for 24h.

After 3 days, LA response of sebocytes is correlated with the presence of "supplement C" in the basal medium (Figure 20).

After 5 days, LA response of sebocytes is correlated with the presence of "supplement A+C" ("Supp A+C") or in the concomitant presence of both "supplement A+C" and "supplement B" ("Supp A+C+B") in the basal medium (Figure 21).

Again, a dose-dependent LA response of sebocytes is correlated with the presence of "supplement A+C" and "supplement B" ("supp A+C+B") in the basal medium (Figure 22).

### Example 7 - DHT response of hiPSC-derived sebocytes cultured sequentially in basal medium complemented with (1) SB431542 and GW0742 ("supplement A+C") and (2) cyclopamine ("supplement B")

Figure 23 illustrates the culture parameters. Sebocyte precursor cells are cultured sequentially in basal medium complemented with supplement A+C from d1 to d3, and in basal medium complemented with supplement B from d3 to d5. At d5 sebocytes are further processed with or without 10 µM DHT and the lipogenesis is assessed after 96h (at d9).

Figure 24 shows that there is a statistically significant response of sebocytes to a DHT treatment, with approximately a 5-fold accumulation of lipids.

The DHT response of sebocytes was assessed at d3, d5 and d6 upon a sequential culture in basal medium complemented with supplement A+C for 3 days, and a culture for 2 extra days in basal medium complemented with either (1) supplement A+C or (2) supplement B (Figure 25). Sebocyte cells obtained after 3, 5 or 6 days are further processed with or without 10 µM DHT for 96 h.

Except at d3 (Figure 26), a statistically significant response to DHT is observed in the different conditions tested (d5, Figure 27; and d6, Figure 28).

### Example 8 - Analysis of the total lipid content of hiPSC-derived sebocytes cultured sequentially in basal medium complemented with (1) SB431542 and GW0742 ("supplement A+C") and (2) cyclopamine ("supplement B"), with or without 10 µM DHT

Figure 29 illustrates the culture parameters. The hiPSC-derived sebocytes are cultured sequentially in basal medium complemented with (1) supplement A+C for 3 days, and (2) supplement B for 3 days, in the absence or the presence of 10 µM DHT. Subsequently, sebocytes obtained after 6 days are analyzed for determining their total lipid content, by the mean of Gaz Chromatography/Mass Spectometry (GC/MS).

It can be observed that hiPSC-derived sebocytes cultured in both conditions are characterized by the presence of sapienic acid, which is naturally found in sebum. This result confirms that hiPSC-derived sebocytes cultured according to the method described herein display a lipid profile similar to *in vitro* cultured primary sebocyte cells (McNairn et al., 2013). Furthermore, the Δ6/Δ9 desaturase ratio, which is an index of sebocyte maturation (Ottaviani et al.; 2010) is close to the ratio observed in sebum obtained from human skin, especially in the sample treated with DHT.

**Table 1 below provides the results of the analysis of lipid markers in both culture conditions:**

| **Lipid marker** | **Supp. A+C+B** | **Supp. A+C+B + 10 µM DHT** |
|---|---|---|
| **Cholesterol** | 64.5 µg/mg | 148.2 µg/mg |
| **Cholesterol esters** | 5.21 µg/mg | 12.45 µg/mg |
| **Triglycerides** | 7.23 µg/mg | 14.77 µg/mg |
| **Sapienic acid** | 0.21% | 1.03% |
| **Palmitoleic acid** | 0.45% | 1.29% |
| **Sapienic acid Δ6/Δ9 ratio** | 46.7 | 79.8 |

Addition of DHT increases lipid accumulation in hiPSC-derived sebocytes, as demonstrated earlier. It also increases maturation of sebocytes, since there is more sapienic acid produced in the presence of DHT, and further increases the Δ6/Δ9 desaturase ratio. The sapienic acid Δ6/Δ9 ratio is about 11 in keratinocytes, while reported at about 180 in primary sebocytes (Mac Nairn, 2013). The ratio of Δ6/Δ9 desaturase measured in hiPSC-derived sebocytes under DHT treatment is therefore clearly above the ratio measured *in vitro* keratinocytes, while under primary sebocytes.

Increased sebocyte maturation upon DHT treatment is in accordance with previous report (Barrault et al.; 2015).

### Example 9 - Characterization of hiPSC-derived sebocytes cultured sequentially for 3 days in basal medium supplemented with SB431542 and GW0742 ("supplement A+C") and for 2 extra days in basal medium supplemented with either supplement A+C (see above) or supplemented with cyclopamine ("supplement B")

hiPSC-derived sebocytes specific markers (MUC1, KRT7, PPARG and FADS2; Figure 31), as well as functional markers (FDFT1, AR, PLIN2, SCD; Figure 32), are expressed following treatment with Supp A+C (A+C) or Supp A+C followed by Supp B (A+C+B) (see Figure 30).

Importantly, the MC5R marker is clearly detected by qPCR in hiPSCs-derived sebocytes, with higher expression following a treatment A+C+B.

It is noteworthy to mention that MC5R marker has been reported to be absent in SZ95 cells by mRNA analysis (Bohm et al.; 2002), whereas MC5R expression in primary keratinocyte has been observed (see Figure 33), as already reported by Hatta et al. (2001). MC5R is a marker of terminally differentiated sebocytes *in vivo* and *in vitro* (Zhang et al.; 2006).

Therefore, the hiPSC-derived sebocytes generated by the methods described herein possess characteristics that are very similar to the *in vitro* culture sebocytes originating from human individuals.

### REFERENCES

Barrault et al. Androgens induce sebaceous differentiation in sebocyte cells expressing a stable functional androgen receptor. J Steroid Biochem Mol Biol. 2015; Aug;152:34-44.
Böhm et al. Evidence for expression of melanocortin-1 receptor in human sebocytes in vitro and in situ. J Invest Dermatol. 2002; Mar;118(3):533-9.
Chen et al. Expression of peroxisome proliferator-activated receptor and CCAAT/enhancer binding protein transcription factors in cultured human sebocytes. J Invest Dermatol. 2003; Sep;121(3):441-7.
Deplewski et al. Unique mode of lipogenic activation in rat preputial sebocytes. J Nutr Metab. 2011;2011:163631.
Downie et al. Modelling the remission of individual acne lesions in vitro. Br J Dermatol. 2002; 147, 869-878.
Fontes et al. Generation of human-induced pluripotent stem cells (hiPSCs) using episomal vectors on defined Essential 8™ Medium conditions. Methods Mol Biol. 2013; 997:57-72.
Hatta et al. Expression, candidate gene, and population studies of the melanocortin 5 receptor. J Invest Dermatol. 2001; Apr;116(4):564-70.
Hou et al. Pluripotent stem cells induced from mouse somatic cells by small-molecule compounds. 2013; Science 341(6146): 651-654.
Makrantonaki et al. An update on the role of the sebaceous gland in the pathogenesis of acne. Dermatoendocrinol. 2011; Jan;3(1):41-9.
McNairn et al. TGFbeta signaling regulates lipogenesis in human sebaceous glands cells. BMC Dermatol. 2013; 13, 2.
Niemann et al. "Indian hedgehog and beta-catenin signaling: role in the sebaceous lineage of normal and neoplastic mammalian epidermis." Proc Natl Acad Sci USA. 2003; 100 Suppl 1: 11873-11880.
Ottaviani et al. Lipid mediators in acne. Mediators Inflamm. 2010;2010.
Pappas. "Epidermal surface lipids." Dermatoendocrinol 2009 ; 1(2): 72-76.
Pochi. "Sebaceous gland assay." In: Lowe N, Maibach H, editors. Models in dermatology. 1985; 2: 70-75.
Soares et al. "International Coordination of Large-Scale Human Induced Pluripotent Stem Cell Initiatives: Wellcome Trust and ISSCR Workshops White Paper." Stem Cell Reports 3.6 (2014): 931-939.
Takahashi and Yamanaka. Induction of pluripotent stem cells from mouse embryonic and adult fibroblast cultures by defined factors. Cell. 2006; 126, 663-676.
Takahashi et al. Induction of pluripotent stem cells from adult human fibroblasts by defined factors. Cell. 2007 ;131(5):861-72.
Toth et al. "Sebocytes' makeup": novel mechanisms and concepts in the physiology of the human sebaceous glands. Pflugers Arch. 2011; 461(6): 593-606.
Trivedi et al. Peroxisome proliferator-activated receptors increase human sebum production. J Invest Dermatol. 2006; Sep;126(9):2002-9.
Xia et al. Culture of human sebocytes in vitro. Dermatoendocrinol. 2009 Mar;1(2):92-5.
Yang et al. Generation of folliculogenic human epithelial stem cells from induced pluripotent stem cells. Nat Commun 2014 ; 5, 3071.
Zhang et al. Melanocortin-5 receptor: a marker of human sebocyte differentiation. Peptides. 2006 Feb;27(2):413-20.
Zouboulis et al. Establishment and characterization of an immortalized human sebaceous gland cell line (SZ95). J Invest Dermatol. 1999; Dec;113(6):1011-20.

## Claims

1. An *in vitro* method for obtaining hiPSC-derived sebocyte precursor cells comprising the steps of:
a) culturing, under feeder layer-free conditions, human induced pluripotent stem cells (hiPSC) in a sebocyte basal medium supplemented with a combination of (i) retinoic acid (RA), (ii) bone morphogenetic protein 4 (BMP4) and (iii) epidermal growth factor (EGF), during a period of time ranging from about 5 days to about 15 days, so as to generate multipotent skin precursor cells,
b) culturing, under feeder layer-free conditions, the multipotent skin precursor cells obtained at step a) in a sebocyte differentiation basal medium, during a period of time ranging from about 5 days to about 15 days, so as to generate hiPSC-derived sebocyte precursor cells, and
c) collecting the hiPSC-derived sebocyte precursor cells obtained at step b).

2. The *in vitro* method according to claim 1, further comprising a step d) of freezing the hiPSC-derived sebocyte precursor cells collected at step c).

3. hiPSC-derived sebocyte precursor cell population obtained by the *in vitro* method according to any one of claims 1 or 2.

4. The hiPSC-derived sebocyte precursor cell population according to claim 3, wherein said population comprises more than 80% of cells expressing KRT7.

5. An *in vitro* method for obtaining hiPSC-derived sebocytes comprising step a) of culturing, under feeder layer-free conditions, hiPSC-derived sebocyte precursor cells obtained by the method according to any one of claim 1 or 2 in a sebocyte basal medium complemented by an inhibitor of the Small Mothers Against Decapentaplegic (SMAD) protein signalling pathway, during a period of time of about 2 days to about 7 days.

6. The *in vitro* method according to claim 5, wherein the said inhibitor of the SMAD protein signaling pathway is 4-(4-(benzo[d][1,3]dioxol-5-yl)-5-(pyridin-2-yl)-1H-imidazol-2-yl)benzamide (SB431542).

7. The *in vitro* method according to any one of claim 5 or 6, wherein the said sebocyte culture medium is further complemented by an agonist of peroxisome proliferator activated receptor (PPAR) delta in the said hiPSC-derived sebocytes.

8. The *in vitro* method according to claim 7, wherein the agonist of peroxisome proliferator activated receptor (PPAR) delta is GW0742.

9. The *in vitro* method according to any one of claims 5 to 8, further comprising the step b) of culturing, under feeder layer-free conditions, the hiPSC-derived sebocytes obtained at step a) in a sebocyte basal medium supplemented by a compound capable of inducing lipogenesis in the said hiPSC-derived sebocytes, during a period of time of about 2 days to about 7 days.

10. The *in vitro* method according to claim 9, wherein the compound capable of inducing lipogenesis is selected in a group comprising a Hedgehog pathway inhibitor.

11. The *in vitro* method according to claim 10, wherein the Hedgehog pathway inhibitor is selected in a group comprising cyclopamine, KAAD-cyclopamine, Jervine, Staurosporinone, Physalin B, Physalin F, Sant 1, Sant 2, Sant 3, Sant 4, Cur-61414, GANT58, GANT61, Robotnikinin, IPI-926 (Saridegib), R51,211 (Itraconazole), and GDC-0449 (Vismodegib).

12. hiPSC-derived sebocytes obtained by an *in vitro* method according to any one of claims 5 to 11.

13. The use of hiPSC-derived sebocyte precursor cells according to claim 4 and/or hiPSC-derived sebocytes according to claim 12, for the preparation of an *in vitro* human model of hypo-seborrheic human skin or hyper-seborrheic human skin.

14. The use of hiPSC-derived sebocyte precursor cells according to claim 4 and/or hiPSC-derived sebocytes according to claim 12, for the preparation of an *in vitro* human model of 3D skin epithelium.

15. The use of hiPSC-derived sebocytes according to claim 12, for the preparation of an *in vitro* human model of testosterone-sensitive cells.

16. The use of hiPSC-derived sebocytes according to claim 12, for the preparation of an *in vitro* human model of linoleic acid-sensitive cells.

17. The use of hiPSC-derived sebocytes according to claim 12, as a tool for screening a compound suitable for the treatment and/or the prevention of the dysfunction of a sebaceous gland.

18. The use of hiPSC-derived sebocytes according to claim 12, as a tool for screening a compound suitable for the treatment and/or the prevention of hypo-seborrheic or hyper-seborrheic conditions of the human skin.
